# EUROPÄISCHE PATENTSCHRIFT

(11) **EP 1 604 688 B1**
(45) Veröffentlichungstag und Bekanntmachung des Hinweises auf die Patenterteilung: **03.02.2010**
(21) Anmeldenummer: 05004938.6
(22) Anmeldetag: 05.06.2002
(51) Int. Cl.: A61K 48/00, A61K 39/00, A61K 39/145, A61P 31/16, A61P 35/04

(54) **Stabilisierte Tumorantigen-mRNA mit erhöhtem G/C-Gehalt**
Stabilised tumour-antigen mRNA with increased G/C-content
ARNm stabilisé codant pour un antigène tumoral avec un contenu G/C augmenté

(30) Priorität: 05.06.2001 DE 10127283
(43) Veröffentlichungstag der Anmeldung: 14.12.2005
(62) Teilanmeldung aus: 02732752.7
(73) Patentinhaber: Curevac GmbH, 72076 Tübingen (DE)
(72) Erfinder: von der Mülbe, Florian, 72070 Tübingen (DE); Pascolo, Steve, 72072 Tübingen (DE); Hoerr, Ingmar, 72070 Tübingen (DE)
(74) Vertreter: Graf von Stosch, Andreas

(56) Entgegenhaltungen:
- EP-A- 1 083 232
- WO-A-01/04313
- WO-A-02/08435
- WO-A-97/48370
- WO-A-98/34640
- WO-A-99/14346
- WO-A-99/20774
- WO-A-03/051401
- US-B1- 6 214 804
- LATHE R: "SYNTHETIC OLIGONUCLEOTIDE PROBES DEDUCED FROM AMINO ACID SEQUENCE DATA. THEORETICAL AND PRACTICAL CONSIDERATIONS" JOURNAL OF MOLECULAR BIOLOGY, LONDON, GB, Bd. 183, Nr. 1, 5. Mai 1985 (1985-05-05), Seiten 1-12, XP000674208 ISSN: 0022-2836
- HOERR INGMAR ET AL: "In vivo application of RNA leads to induction of specific cytotoxic T lymphocytes and antibodies" EUROPEAN JOURNAL OF IMMUNOLOGY, Bd. 30, Nr. 1, Januar 2000 (2000-01), Seiten 1-7, XP002243972 ISSN: 0014-2980
- HOLCIK M ET AL: "Four highly stable eukaryotic mRNAs assemble 3' untranslated region RNA-protein complexes sharing cis and trans components" PROCEEDINGS OF THE NATIONAL ACADEMY OF SCIENCES OF USA, NATIONAL ACADEMY OF SCIENCE, WASHINGTON, DC, US, Bd. 94, Nr. 6, 1997, Seiten 2410-2414, XP002243974 ISSN: 0027-8424

### SEQUENCE LISTING

<110> Von der Muelbe, Florian
   Hoerr, Ingmar (nur US)
   Pascolo, Steve (nur US)
<120> Pharmazeutische Zusammensetzung, enthaltend eine stabilisierte und für die Translation in ihren codierenden Bereichen optimierte mRNA
<130> CU01P003WO
<140> PCT/EP02/06180
   <141> 2002-06-05
<160> 13
<170> PatentIn Ver. 2.1
<210> 1
   <211> 774
   <212> DNA
   <213> Influenza virus
<220>
   <223> Influenza-Matrix: Wildtyp-Gen (zum Vergleich)
<220>
   <223> Startcodon: atg (Nucleotide 11 bis 13), Stopcodon: tga (Nucleotide 767 bis 769 )
<400> 1
   agatctaaag atgagtcttc taaccgaggt cgaaacgtac gttctctcta tcatcccgtc 60
   aggccccctc aaagccgaga tcgcacagag acttgaagat gtctttgcag ggaagaacac 120
   cgatcttgag gttctcatgg aatggctaaa gacaagacca atcctgtcac ctctgactaa 180
   ggggatttta ggatttgtgt tcacgctcac cgtgcccagt gagcgaggac tgcagcgtag 240
   acgctttgtc caaaatgccc ttaatgggaa cggggatcca aataacatgg acaaagcagt 300
   taaactgtat aggaagctca agagggagat aacattccat ggggccaaag aaatctcact 360
   cagttattct gctggtgcac ttgccagttg tatgggcctc atatacaaca ggatgggggc 420
   tgtgaccact gaagtggcat ttggcctggt atgtgcaacc tgtgaacaga ttgctgactc 480
   ccagcatcgg tctcataggc aaatggtgac aacaaccaac ccactaatca gacatgagaa 540
   cagaatggtt ttagccagca ctacagctaa ggctatggag caaatggctg gatcgagtga 600
   gcaagcagca gaggccatgg aggttgctag tcaggctagg caaatggtgc aagcgatgag 660
   aaccattggg actcatccta gctccagtgc tggtctgaaa aatgatcttc ttgaaaattt 720
   gcaggcctat cagaaacgaa tgggggtgca gatgcaacgg ttcaagtgaa ctag 774
<210> 2
   <211> 252
   <212> PRT
   <213> Influenza virus
<400> 2
   Met Ser Leu Leu Thr Glu Val Glu Thr Tyr Val Leu Ser Ile Ile Pro
   1 5 10 15
   Ser Gly Pro Leu Lys Ala Glu Ile Ala Gln Arg Leu Glu Asp Val Phe
   20 25 30
   Ala Gly Lys Asn Thr Asp Leu Glu Val Leu Met Glu Trp Leu Lys Thr
   35 40 45
   Arg Pro Ile Leu Ser Pro Leu Thr Lys Gly Ile Leu Gly Phe Val Phe
   50 55 60
   Thr Leu Thr Val Pro Ser Glu Arg Gly Leu Gln Arg Arg Arg Phe Val
   65 70 75 80
   Gln Asn Ala Leu Asn Gly Asn Gly Asp Pro Asn Asn Met Asp Lys Ala
   85 90 95
   Val Lys Leu Tyr Arg Lys Leu Lys Arg Glu Ile Thr Phe His Gly Ala
   100 105 110
   Lys Glu Ile Ser Leu Ser Tyr Ser Ala Gly Ala Leu Ala Ser Cys Met
   115 120 125
   Gly Leu Ile Tyr Asn Arg Met Gly Ala Val Thr Thr Glu Val Ala Phe
   130 135 140
   Gly Leu Val Cys Ala Thr Cys Glu Gln Ile Ala Asp Ser Gln His Arg
   145 150 155 160
   Ser His Arg Gln Met Val Thr Thr Thr Asn Pro Leu Ile Arg His Glu
   165 170 175
   Asn Arg Met Val Leu Ala Ser Thr Thr Ala Lys Ala Met Glu Gln Met
   180 185 190
   Ala Gly Ser Ser Glu Gln Ala Ala Glu Ala Met Glu Val Ala Ser Gln
   195 200 205
   Ala Arg Gln Met Val Gln Ala Met Arg Thr Ile Gly Thr His Pro Ser
   210 215 220
   Ser Ser Ala Gly Leu Lys Asn Asp Leu Leu Glu Asn Leu Gln Ala Tyr
   225 230 235 240
   Gln Lys Arg Met Gly Val Gln Met Gln Arg Phe Lys
   245 250
<210> 3
   <211> 775
   <212> DNA
   <213> Artificial Sequence
<220>
   <223> Description of Artificial Sequence: Influenza-Matrix: Gen mit erhöhtem G/C-Gehalt
<220>
   <223> Startcodon: atg (Nucleotide 11 bis 13), Stopcodon: tga (Nucleotide 767 bis 769)
<400> 3
   agatctaaag atgagcctgc tgaccgaggt ggagacctac gtgctgagca tcatccccag 60
   cggccccctg aaggccgaga tcgcccagag gctggaggac gtgttcgccg gcaagaacac 120
   cgacctggag gtgctgatgg agtggctgaa gaccaggccc atcctgagcc ccctgaccaa 180
   gggcatcctg ggcttcgtgt tcaccctgac cgtgcccagc gagcgcggcc tgcagcgccg 240
   ccgcttcgtg cagaacgccc tgaacggcaa cggcgacccc aacaacatgg acaaggccgt 300
   gaagctgtac aggaagctga agagggagat caccttccac ggcgccaagg agatcagcct 360
   gagctacagc gccggcgccc tggccagctg catgggcctg atctacaaca ggatgggcgc 420
   cgtgaccacc gaggtggcct tcggcctggt gtgcgccacc tgcgagcaga tcgccgacag 480
   ccagcaccgc agccacaggc agatggtgac caccaccaac cccctgatca ggcacgagaa 540
   caggatggtg ctggccagca ccaccgccaa ggccatggag cagatggccg gcagcagcga 600
   gcaggccgcc gaggccatgg aggtggccag ccaggccagg cagatggtgc aggccatgag 660
   gaccatcggc acccacccca gcagcagcgc cggcctgaag aacgacctgc tggagaacct 720
   gcaggcctac cagaagcgca tgggcgtgca gatgcagcgc ttcaagtgaa ctagt      775
<210> 4
   <211> 844
   <212> DNA
   <213> Artificial Sequence
<220>
   <223> Description of Artificial Sequence: Influenza-Matrix: Gen für sekretierte Form (mit N-terminaler Signalsequenz) mit erhöhtem G/C-Gehalt
<220>
   <223> Startcodon: atg (Nucleotide 11 bis 13), Stopcodon: tga (Nucleotide 836 bis 838)
<400> 4
   agatctaaag atggccgtca tggccccccg caccctggtg ctgctgctga gcggcgccct 60
   ggccctgacc cagacctggg ctagcctgct gaccgaggtg gagacctacg tgctgagcat 120
   catccccagc ggccccctga aggccgagat cgcccagagg ctggaggacg tgttcgccgg 180
   caagaacacc gacctggagg tgctgatgga gtggctgaag accaggccca tcctgagccc 240
   cctgaccaag ggcatcctgg gcttcgtgtt caccctgacc gtgcccagcg agcgcggcct 300
   gcagcgccgc cgcttcgtgc agaacgccct gaacggcaac ggcgacccca acaacatgga 360
   caaggccgtg aagctgtaca ggaagctgaa gagggagatc accttccacg gcgccaagga 420
   gatcagcctg agctacagcg ccggcgccct ggccagctgc atgggcctga tctacaacag 480
   gatgggcgcc gtgaccaccg aggtggcctt cggcctggtg tgcgccacct gcgagcagat 540
   cgccgacagc cagcaccgca gccacaggca gatggtgacc accaccaacc ccctgatcag 600
   gcacgagaac aggatggtgc tggccagcac caccgccaag gccatggagc agatggccgg 660
   cagcagcgag caggccgccg aggccatgga ggtggccagc caggccaggc agatggtgca 720
   ggccatgagg accatcggca cccaccccag cagcagcgcc ggcctgaaga acgacctgct 780
   ggagaacctg caggcctacc agaagcgcat gggcgtgcag atgcagcgct tcaagtgaac 840
   tagt                                                              844
<210> 5
   <211> 942
   <212> RNA
   <213> Artificial Sequence
<220>
   <223> Description of Artificial Sequence: Influenza-Matrix: MRNA mit Stabilisierungssequenzen
<220>
   <223> Die Stabilisierungssequenzen stammen aus den 5'- bzw. 3'-UTRs der B-Globin-mRM von Xenopus laevis
<220>
   <223> Startcodon: aug (Nucleotide 56 bis 58), Stopcodon: uga (Nucleotide 812 bis 814)
<400> 5
<210> 6
   <211> 942
   <212> RNA
   <213> Artificial Sequence
<220>
   <223> Description of Artificial Sequence: Influenza-Matrix: mRNA mit erhöhtem G/C-Gehalt und Stabilisierungssequenzen
<220>
   <223> Die Stabilisierungssequenzen stammen aus den 5'- bzw. 3'-UTRs der B-Globin-mRNA von Xenopus laevis
<220>
   <223> Startcodon: aug (Nucleotide 56 bis 58), Stopcodon: uga (Nucleotide 812 bis 814)
<400> 6
   gcuuguucuu uuugcagaag cucagaauaa acgcucaacu uuggcagauc uaaagaugag 60
   ccugcugacc gagguggaga ccuacgugcu gagcaucauc cccagcggcc cccugaaggc 120
   cgagaucgcc cagaggcugg aggacguguu cgccggcaag aacaccgacc uggaggugcu 180
   gauggagugg cugaagacca ggcccauccu gagcccccug accaagggca uccugggcuu 240
   cguguucacc cugaccgugc ccagcgagcg cggccugcag cgccgccgcu ucgugcagaa 300
   cgcccugaac ggcaacggcg accccaacaa cauggacaag gccgugaagc uguacaggaa 360
   gcugaagagg gagaucaccu uccacggcgc caaggagauc agccugagcu acagcgccgg 420
   cgcccuggcc agcugcaugg gccugaucua caacaggaug ggcgccguga ccaccgaggu 480
   ggccuucggc cuggugugcg ccaccugcga gcagaucgcc gacagccagc accgcagcca 540
   caggcagaug gugaccacca ccaacccccu gaucaggcac gagaacagga uggugcuggc 600
   cagcaccacc gccaaggcca uggagcagau ggccggcagc agcgagcagg ccgccgaggc 660
   cauggaggug gccagccagg ccaggcagau ggugcaggcc augaggacca ucggcaccca 720
   ccccagcagc agcgccggcc ugaagaacga ccugcuggag aaccugcagg ccuaccagaa 780
   gcgcaugggc gugcagaugc agcgcuucaa gugaacuagu gacugacuag cccgcugggc 840
   cucccaacgg gcccuccucc ccuccuugca ccaaaaaaaa aaaaaaaaaa aaaaaaaaaa 900
   aaaaaaaaaa aaaaaaaaaa aaaaaaaaaa aaaaaaaaaa aa                    942
<210> 7
   <211> 1011
   <212> RNA
   <213> Artificial Sequence
<220>
   <223> Description of Artificial Sequence: Influenza-Matrix: für sekretierte Form codierende mRNA mit erhöhtem G/C-Gehalt und Stabilisierungssequenzen
<220>
   <223> startcodon: aug (Nucleotide 56 bis 58), Stopcodon: uga (Nucleotide 881 bis 883)
<400> 7
   gcuuguucuu uuugcagaag cucagaauaa acgcucaacu uuggcagauc uaaagauggc 60
   cgucauggcc ccccgcaccc uggugcugcu gcugagcggc gcccuggccc ugacccagac 120
   cugggccagc cugcugaccg agguggagac cuacgugcug agcaucaucc ccagcggccc 180
   ccugaaggcc gagaucgccc agaggcugga ggacguguuc gccggcaaga acaccgaccu 240
   ggaggugcug auggaguggc ugaagaccag gcccauccug agcccccuga ccaagggcau 300
   ccugggcuuc guguucaccc ugaccgugcc cagcgagcgc ggccugcagc gccgccgcuu 360
   cgugcagaac gcccugaacg gcaacggcga ccccaacaac auggacaagg ccgugaagcu 420
   guacaggaag cugaagaggg agaucaccuu ccacggcgcc aaggagauca gccugagcua 480
   cagcgccggc gcccuggcca gcugcauggg ccugaucuac aacaggaugg gcgccgugac 540
   caccgaggug gccuucggcc uggugugcgc caccugcgag cagaucgccg acagccagca 600
   ccgcagccac aggcagaugg ugaccaccac caacccccug aucaggcacg agaacaggau 660
   ggugcuggcc agcaccaccg ccaaggccau ggagcagaug gccggcagca gcgagcaggc 720
   cgccgaggcc auggaggugg ccagccaggc caggcagaug gugcaggcca ugaggaccau 780
   cggcacccac cccagcagca gcgccggccu gaagaacgac cugcuggaga accugcaggc 840
   cuaccagaag cgcaugggcg ugcagaugca gcgcuucaag ugaacuagug acugacuagc 900
   ccgcugggcc ucccaacggg cccuccuccc cuccuugcac caaaaaaaaa aaaaaaaaaa 960
   aaaaaaaaaa aaaaaaaaaa aaaaaaaaaa aaaaaaaaaa aaaaaaaaaa a          1011
<210> 8
   <211> 940
   <212> DNA
   <213> Homo sapiens
<220>
   <223> MAGEl: Wildtyp-Gen (zum Vergleich)
<220>
   <223> Startcodon: atg (Nucleotide 5 bis 7), Stopcodon: tga (Nucleotide 932 bis 934)
<400> 8
   catcatgtct cttgagcaga ggagtctgca ctgcaagcct gaggaagccc ttgaggccca 60
   acaagaggcc ctgggcctgg tgtgtgtgca ggctgccacc tcctcctcct ctcctctggt 120
   cctgggcacc ctggaggagg tgcccactgc tgggtcaaca gatcctcccc agagtcctca 180
   gggagcctcc gcctttccca ctaccatcaa cttcactcga cagaggcaac ccagtgaggg 240
   ttccagcagc cgtgaagagg aggggccaag cacctcttgt atcctggagt ccttgttccg 300
   agcagtaatc actaagaagg tggctgattt ggttggtttt ctgctcctca aatatcgagc 360
   cagggagcca gtcacaaagg cagaaatgct ggagagtgtc atcaaaaatt acaagcactg 420
   ttttcctgag atcttcggca aagcctctga gtccttgcag ctggtctttg gcattgacgt 480
   gaaggaagca gaccccaccg gccactccta tgtccttgtc acctgcctag gtctctccta 540
   tgatggcctg ctgggtgata atcagatcat gcccaagaca ggcttcctga taattgtcct 600
   ggtcatgatt gcaatggagg gcggccatgc tcctgaggag gaaatctggg aggagctgag 660
   tgtgatggag gtgtatgatg ggagggagca cagtgcctat ggggagccca ggaagctgct 720
   cacccaagat ttggtgcagg aaaagtacct ggagtaccgg caggtgccgg acagtgatcc 780
   cgcacgctat gagttcctgt ggggtccaag ggccctcgct gaaaccagct atgtgaaagt 840
   ccttgagtat gtgatcaagg tcagtgcaag agttcgcttt ttcttcccat ccctgcgtga 900
   agcagctttg agagaggagg aagagggagt ctgagcatga                       940
<210> 9
   <211> 308
   <212> PRT
   <213> Homo sapiens
<220>
   <223> Tumorantigen HAGEl: Proteinsequenz
<900> 9
<210> 10
   <211> 939
   <212> RNA
   <213> Artificial Sequence
<220>
   <223> Description of Artificial Sequence: MAGE1: mRNA mit erhöhtem G/C-Gehalt
<220>
   <223> Startcodon: aug (Nucleotide 1 bis 3), Stopcodon: uga (Nucleotide 937 bis 939)
<400> 10
   augagccugg agcagcgcag ccugcacugc aagccggagg aggcgcugga ggcgcagcag 60
   gaggcgcugg gccuggucug cguccaggcg gcgacgagca gcagcagccc gcugguccug 120
   ggcacgcugg aggaggaccc gacggcgggc agcacggacc cgccgcagag cccgcagggc 180
   gcgagcgcgu ucccgacgac gaucaacuuc acgcgccagc gccagccgag cgagggcagc 240
   agcagccgcg aggaggaggg cccgagcacg agcugcaucc uggagagccu guuccgcgcg 300
   gucaucacga agaaggucgc ggaccugguc ggcuuccugc ugcugaagua ccgcgcgcgc 360
   gagccgguca cgaaggcgga gaugcuggag agcgucauca agaacuacaa gcacugcuuc 420
   ccggagaucu ucggcaaggc gagcgagagc cugcagcugg ucuucggcau cgacgucaag 480
   gaggcggacc cgacgggcca cagcuacguc cuggucacgu gccugggccu gagcuacgac 540
   ggccugcugg gcgacaacca gaucaugccg aagacgggcu uccugaucau cguccugguc 600
   augaucgcga uggagggcgg ccacgcgccg gaggaggaga ucugggagga gcugagcguc 660
   auggaggucu acgacggccg cgagcacagc gcguacggcg agccgcgcaa gcugcugacg 720
   caggaccugg uccaggagaa guaccuggag uaccgccagg ucccggacag cgacccggcg 780
   cgcuacgagu uccugugggg cccgcgcgcg cuggcggaga cgagcuacgu caagguccug 840
   gaguacguca ucaaggucag cgcgcgcguc cgcuucuucu ucccgagccu gcgcgaggcg 900
   gcgcugcgcg aggaggagga gggcgucuga gcgugauga                        939
<210> 11
   <211> 939
   <212> RNA
   <213> Artificial Sequence
<220>
   <223> Description of Artificial Sequence: MAGE1: mRNA mit alternativer Codonverwendung
<220>
   <223> Startcodon: aug (Nucleotide 1 bis 3), Stopcodon: uga (Nucleotide 937 bis 939)
<400> 11
   augagccugg agcagcgcag ccugcacugc aagcccgagg aggcccugga ggcccagcag 60
   gaggcccugg gccuggugug cgugcaggcc gccaccagca gcagcagccc ccuggugcug 120
   ggcacccugg aggaggugcc caccgccggc agcaccgacc ccccccagag cccccagggc 180
   gccagcgccu uccccaccac caucaacuuc acccgccagc gccagcccag cgagggcagc 240
   agcagccgcg aggaggaggg ccccagcacc agcugcaucc uggagagccu guuccgcgcc 300
   gugaucacca agaagguggc cgaccuggug ggcuuccugc ugcugaagua ccgcgcccgc 360
   gagcccguga ccaaggccga gaugcuggag agcgugauca agaacuacaa gcacugcuuc 420
   cccgagaucu ucggcaaggc cagcgagagc cugcagcugg uguucggcau cgacgugaag 480
   gaggccgacc ccaccggcca cagcuacgug cuggugaccu gccugggccu gagcuacgac 540
   ggccugcugg gcgacaacca gaucaugccc aagaccggcu uccugaucau cgugcuggug 600
   augaucgcca uggagggcgg ccacgccccc gaggaggaga ucugggagga gcugagcgug 660
   auggaggugu acgacggccg cgagcacagc gccuacggcg agccccgcaa gcugcugacc 720
   caggaccugg ugcaggagaa guaccuggag uaccgccagg ugcccgacag cgaccccgcc 780
   cgcuacgagu uccugugggg cccccgcgcc cuggccgaga ccagcuacgu gaaggugcug 840
   gaguacguga ucaaggugag cgcccgcgug cgcuucuucu uccccagccu gcgcgaggcc 900
   gcccugcgcg aggaggagga gggcguguga gccugauga                        939
<210> 12
   <211> 7
   <212> RNA
   <213> Artificial Sequence
<220>
   <223> Description of Artificial Sequence: Ein für eine Endonuklease erkennbares Sequenzmotiv, das im 3'UTR-Segment des für den Transferin-Rezeptor codierenden Gens enthalten ist (S. 10 der Beschreibung).
<400> 12
   gaacaag 7
<210> 13
   <211> 13
   <212> RNA
   <213> Artificial Sequence
<220>
   <223> Description of Artificial Sequence: Kozak-Sequenz, Ribosomen-Bindungsstelle (S. 12 der Beschreibung)
<400> 13
   gccgccacca ugg 13

## Beschreibung

Die vorliegende Erfindung betrifft eine pharmazeutische Zusammensetzung, enthaltend eine mRNA, die durch Sequenzmodifikationen im translatierten Bereich stabilisiert und für die Translation optimiert ist sowie derartige stabilisierte in RNA. Die erfindungsgemäße pharmazeutische Zusammensetzung eignet sich insbesondere als Impfstoff.

Die Gentherapie und die genetische Vakzinierung sind molekularmedizinische Verfahren, deren Anwendung in der Therapie und Prävention von Erkrankungen erhebliche Auswirkungen auf die medizinische Praxis haben wird. Beide Verfahren beruhen auf der Einbringung von Nukleinsäuren in Zellen bzw. in Gewebe des Patienten sowie auf der anschließenden Verarbeitung der durch die eingebrachten Nukleinsäuren codierten Information, d.h. der Expression der erwünschten Polypeptide.

Die übliche Vorgehensweise bisheriger Verfahren der Gentherapie und der genetischen Vakzinierung ist die Verwendung von DNA, um die benötigte genetische Information in die Zelle einzuschleusen. In diesem Zusammenhang sind verschiedene Verfahren zur Einbringung von DNA in Zellen, wie bspw. Calciumphosphat-Transfektion, Polypren-Transfektion, Protoplasten-Fusion, Elektroporation, Mikroinjektion und Lipofektion, entwickelt worden, wobei sich insbesondere die Lipofektion als geeignetes Verfahren herausgestellt hat.

Ein weiteres Verfahren, das insbesondere bei genetischen Vakzinierungsverfahren vorgeschlagen wurde, ist die Verwendung von DNA-Viren als DNA-Vehikel. Derartige Viren haben den Vorteil, daß aufgrund ihrer infektiösen Eigenschaften eine sehr hohe Transfektionsrate zu erzielen ist Die verwendeten Viren werden genetisch verändert, so daß in der transfizierten Zelle keine funktionsfähigen infektiöse Partikel gebildet werden. Trotz dieser Vorsichtsmaßnahme kann jedoch aufgrund möglicher Rekombinationsereignisse ein gewisses Risiko der unkontrollierten Ausbreitung der eingebrachten gentherapeutisch wirksamen sowie viralen Gene nicht ausgeschlossen werden.

Üblicherweise wird die in die Zelle eingebrachte DNA in gewissem Ausmaß in das Genom der transfizierten Zelle integriert Einerseits kann dieses Phänomen einen erwünschten Effekt ausüben, da hierdurch eine langandauernde Wirkung der eingebrachten DNA erzielt werden kann. Andererseits bringt die Integration in das Genom ein wesentliches Risiko der Gentherapie mit sich. So kann es bspw. zu einer Insertion der eingebrachten DNA in ein intaktes Gen kommen, was eine Mutation darstellt, welche die Funktion des endogen Gens behindert oder gar vollkommen ausschaltet. Durch solche Integrationsereignisse können einerseits für die Zelle lebenswichtige Enzymsystem ausgeschaltet werden, andererseits besteht auch die Gefahr einer Transformation der so veränderten Zelle in einen entarteten Zustand, falls durch die Integration der Fremd-DNA ein für die Regulation des Zellwachstums entscheidende Gen verändert wird. Daher kann bei der Verwendung von DNA-Viren als Gentherapeutika und Vakzine ein Risiko der Krebsbildung nicht ausgeschlossen werden. In diesem Zusammenhang ist auch zu beachten, daß zur wirksamen Expression der in die Zelle eingebrachten Gene die entsprechenden DNA-Vehikel einen starken Promotor, bspw. den viralen CMV-Promotor, enthalten. Die Integration derartiger Promotoren in das Genom der behandelten Zelle kann zu unerwünschten Veränderungen der Regulierung der Genexpression in der Zelle führen.

Ein weiterer Nachteil der Verwendung von DNA als Gentherapeutika und Vakzine ist die Induktion pathogener Anti-DNA-Antikörper im Patienten unter Hervorrufung einer möglicherweise tödlichen Immunantwort.

Im Gegensatz zu DNA ist der Einsatz von RNA als Gentherapeutikum oder Vakzin als wesentlich sicherer einzustufen. Insbesondere bringt RNA nicht die Gefahr mit sich, stabil in das Genom der transfizierten Zelle integriert zu werden. Des weiteren sind keine viralen Sequenzen, wie Promotoren, zur wirksamen Transkription, erforderlich. Darüber hinaus wird RNA wesentlich einfacher *in vivo* abgebaut. Wohl aufgrund der gegenüber DNA relativ kurzen Halbwertszeit von RNA im Blutkreislauf sind bisher keine anti-RNA-Antikörper nachgewiesen worden. Daher kann RNA für molekularmedizinische Therapie-Verfahren als Molekül der Wahl angesehen werden.

Allerdings bedürfen auf RNA-Expressionssystemen beruhende medizinische Verfahren vor einer breiteren Anwendung noch der Lösung einiger grundsätzlicher Probleme. Eines der Probleme bei der Verwendung von RNA eist der sichere, Zell- bzw. Gewebespezifische effiziente Transfer der Nukleinsäure. Da sich RNA in Lösung normalerweise als sehr instabil erweist, konnte durch die herkömmlichen Verfahren, die bei DNA verwendet werden, RNA bisher nicht oder nur sehr ineffizient als Therapeutikum bzw. Impfstoff verwendet werden.

Für die Instabilität sind RNA-abbauende Enzyme, sog. RNAasen (Ribonucleasen), verantwortlich. Selbst kleinste Verunreinigungen von Ribonucleasen reichen aus, um RNA in Lösung vollständig abzubauen. Der natürliche Abbau von mRNA im Cytoplasma von Zellen ist sehr fein reguliert. Diesbezüglich sind mehrere Mechanismen bekannt So ist für eine funktionale mRNA die endständige Struktur von entscheidender Bedeutung. Am 5'-Ende befindet sich die sogenannte "Cap-Struktur" (ein modifiziertes Guanosin-Nucleotid) und am 3'-Ende eine Abfolge von bis zu 200 Adenosin-Nucleotiden (der sog. Poly-A-Schwanz). Über diese Strukturen wird die RNA als mRNA erkannt und der Abbau reguliert. Darüber hinaus gibt es weitere Prozesse, die RNA stabilisieren bzw. destabilisieren. Viele diese Prozesse sind noch unbekannt, oftmals scheint jedoch eine Wechselwirkung zwischen der RNA und Proteinen dafür maßgeblich zu sein. Bspw. wurde kürzlich ein "mRNA-Surveillance-System" beschrieben (Hellerin und Parker, Annu. Rev. Genet. 1999, 33: 229 bis 260), bei dem durch bestimmte Feedback-Protein-Wechselwirkungen im Cytosol unvollständige oder Nonsense-mRNA erkannt und dem Abbau zugänglich gemacht wird, wobei ein Hauptteil dieser Prozesse durch Exonucleasen vollzogen wird.

Im Stand der Technik sind einige Maßnahmen vorgeschlagen worden, um die Stabilität von RNA zu erhöhen und dadurch ihren Einsatz als Gentherapeutikum bzw. RNA-Vakzine zu ermöglichen.

In diesem Zusammenhang beschreibt WO 98/34640 jedoch nur synthetische DNA Moleküle, die HIV gag und Modifikationen von HIV gag kodieren. Die in WO 98/34640 offenbarten Moleküle können als Polynukleotid-Vakzine eingesetzt werden, die eine effektive Immunprophylaxe gegen HIV durch Stimulierung neutralisierender Antikörper und Zellvermittelter Immunität ermöglichen. WO 98/34640 beschreibt in diesem Zusammenhang jedoch keine Erhöhungen oder gar Maximierungen des G/C-Gehaltes der kodierenden synthetischen DNA Moleküle und ebenfalls keine in diesem Zusammenhang verwendeten und modifizierten mRNA-Moleküle.

Auch WO 97/48370 offenbart zwar synthetische DNA-Moleküle, die ein Peptid oder Protein kodieren, insbesondere HIV env als auch Modifikationen on HIV env. WO 97/48370 beschreibt aber auch keinerlei Erhöhungen oder gar Maximierungen des G/C-Gehaltes der kodierenden synthetischen DNA Moleküle und ebenfalls keine Modifikationen der in diesem Zusammenhang verwendeten mRNA-Moleküle.

In EP-A-1083232 wird zur Lösung des vorstehend genannten Problems der Instabilität von RNA ex *vivo* ein Verfahren zur Einbringung von RNA, insbesondere mRNA, in Zellen und Organismen vorgeschlagen, bei welchem die RNA in Form eines Komplexes mit einem kationischen Peptid oder Protein vorliegt. Hierbei wird auch offenbart, derartige Komplexe zur Krebstherapie einzusetzen, wobei die mRNA in einem solchen Fall für Tumorantigene codiert.

WO 99/14346 beschreibt weitere Verfahren zur Stabilisierung von mRNA. Insbesondere werden Modifizierungen der mRNA vorgeschlagen, welche die mRNA-Spezies gegenüber dem Abbau von RNasen stabilisieren. Derartige Modifikationen betroffen einerseits die Stabilisierung durch Sequenzmodifikationen, insbesondere Verminderung des C-und/oder U-Gehalts durch Baseneliminierung oder Basensubstitution. Andererseits werden chemische Modifikationen, insbesondere die Verwendung von Nucleotidanaloga, sowie 5'- und 3'- Blockierungsgruppen, eine erhöhte Länge des Poly-A-Schwanzes sowie die Komplexierung der mRNA mit stabilisierenden Mitteln und Kombinationen der genannten Maßnahmen vorgeschlagen.

In den US-Patenten US 5,580,859 und US 6,214,804 werden unter anderem im Rahmen der "transienten Gentherapie" (TGT) mRNA-Vakzine und -Therapeutika offenbart. Es werden verschiedene Maßnahmen zur Erhöhung der Translationseffizienz und der mRNA-Stabilität beschrieben, die sich vor allem auf die nicht-translatierten Sequenzbereiche beziehen.

Bieler und Wagner (in: Schleef (Hrsg.), Plasmids for Therapy and Vaccination, Kapitel 9, Seiten 147 bis 168, Wiley-VCH, Weinheim, 2001) berichten von der Verwendung synthetischer Gene im Zusammenhang mit gentherapeutischen Methoden unter Verwendung von DNA-Vakzinen und lentiveralen Vektoren. Es wird die Konstruktion eines synthetischen, von HIV-1 abgeleiteten *gag*-Gens beschrieben, bei welchem die Codons gegenüber der Wildtyp-Sequenz derart modifiziert wurden (alternative Codonverwendung, engl. "codon usage"), daß sie der Verwendung von Codons entsprach, die in hoch exprimierten Säugergenen zu finden ist. Dadurch wurde insbesondere der A/T-Gehalt gegenüber der Wildtyp-Sequenz vermindert. Die Autoren stellen insbesondere eine erhöhte Expressionsrate des synthetischen *gag*-Gens in transfizierten Zellen fest. Des weiteren wurde in Mäusen eine erhöhte Antikörperbildung gegen das *gag-*Protein bei mit dem synthetischen DNA-Konstrukt immunisierten Mäusen und auch eine verstärkte Cytokinfreisetzung *in vitro* bei transfizierten Milzzellen von Mäusen beobachtet. Schließlich konnte eine Induzierung einer cytotoxischen Immunantwort in mit dem *gag*-Expressionsplasmid immunisierten Mäusen festgestellt werden. Die Autoren dieses Artikels fuhren die verbesserten Eigenschaften ihres DNA-Vakzins im wesentlichen auf einen durch die optimierte Codonverwendung hervorgerufene Veränderung des nucleo-cytoplasmatischen Transports der vom DNA-Vakzin exprimierten mRNA zurück. Im Gegensatz dazu halten die Autoren die Auswirkung der veränderten Codonverwendung auf die Translationseffizienz für gering.

Der vorliegenden Erfindung liegt daher die Aufgabe zugrunde, ein neues System zur genetischen Vakzinierung bereitzustellen, das die mit den Eigenschaften von DNA-Therapeutika und -Vakzinen verbundenen Nachteile überwindet und die Wirksamkeit von auf RNA-Spezies basierenden Therapeutika erhöht.

Diese Aufgabe wird durch die in den Ansprüchen gekennzeichneten Ausführungsformen der vorliegenden Erfindung gelöst.

Insbesondere wird erfindungsgemäß eine modifizierte mRNA sowie mindestens eine derartige modifizierte mRNA in Verbindung mit einem pharmazeutisch verträglichen Träger und/oder Vehikel enthaltende pharmazeutische Zusammensetzung bereitgestellt, wobei die modifizierte erfindungsgemäße mRNA für mindestens ein Tumor-antigenes Peptid oder Polypeptid codiert, wobei die Sequenz der mRNA, insbesondere im für das mindestens eine Peptid oder Polypeptid codierenden Bereich, gegenüber der Wildtyp-mRNA folgende Modifikation aufweist.

So ist der G/C-Gehalt des für das Peptid oder Polypeptid codierenden Bereichs der modifizierten erfindungsgemäßen mRNA größer als der G/C-Gehalt des codierenden Bereichs der für das Peptid oder Polypeptid codierenden Wildtyp-mRNA, wobei die codierte Aminosäuresequenz gegenüber dem Wildtyp unverändert ist.

Diese Modifikation beruht auf der Tatsache, daß für eine effiziente Translation einer mRNA die Sequenzabfolge des zu translatierenden Bereichs der mRNA wesentlich ist. Dabei spielt die Zusammensetzung und die Abfolge der verschiedenen Nucleotide eine große Rolle. Insbesondere sind Sequenzen mit erhöhtem G(Guanosin)/C(Cytosin)-Gehalt stabiler als Sequenzen mit einem erhöhten A(Adenosin)/U(Uracil)-Gehalt. Daher werden erfindungsgemäß unter Beibehaltung der translatierten Aminosäureabfolge die Codons gegenüber der Wildtyp-mRNA derart variiert daß sie vermehrt G/C-Nucleotide beinhalten. Da mehrere Codons für ein und dieselbe Aminosäure codieren (Degeneration des genetischen Codes), können die für die Stabilität günstigsten Codons ermittelt werden (alternative Codonverwendung, engl. "codon usage").

In Abhängigkeit von der durch die modifizierte erfindungsgemäße mRNA zu codierenden Aminosäure sind unterschiedliche Möglichkeiten zur Modifikation der mRNA-Sequenz gegenüber der Wildtyp-Sequenz möglich. Im Fall von Aminosäuren, die durch Codons codiert werden, die ausschließlich G- oder C- Nucleotide enthalten, ist keine Modifikation des Codons erforderlich. So erfordern die Codons für Pro (CCC oder CCG), Arg (CGC oder CGG), Ala (GCC oder GCG) und Gly (GGC oder GGG) keine Veränderung, da kein A oder U vorhanden ist.

In folgenden Fällen werden die Codons, welche A-und/oder U-Nucleotide enthalten durch Substituieren anderer Codons, welche die gleichen Aminosäuren codieren, jedoch kein A und/oder U enthalten, verändert. Beispiele sind:
Die Codons für Pro können von CCU oder CCA zu CCC oder CCG verändert werden;
die Codons für Arg können von CGU oder CGA oder AGA oder AGG zu CGC oder CGG verändert werden;
die Codons für Ala können von GCU oder GCA zu GCC oder GCG verändert werden;
die Codons für Gly können von GGU oder GGA zu GGC oder GGG verändert werden.

In anderen Fällen können A bzw. U-Nucleotide zwar nicht aus den Codons eliminiert werden, es ist jedoch möglich, den A- und U-Gehalt durch Verwendung von Codons zu vermindern, die weniger A- und/oder U-Nucleotide enthalten. Zum Beispiele:
Die Codons für Phe können von UUU zu UUC verändert werden;
die Codons für Leu können von UUA, CUU oder CUA zu CUC oder CUG verändert werden;
die Codons für Ser können von UCU oder UCA oder AGU zu UCC, UCG oder AGC verändert werden;
das Codon für Tyr kann von UAU zu UAC verändert werden;
das Stop-Codon UAA kann zu UAG oder UGA verändert werden;
das Codon für Cys kann von UGU zu UGC verändert werden;
das Codon His kann von CAU zu CAC verändert werden;
das Codon für Gln kann von CAA zu CAG verändert werden;
die Codons für Ile können von AUU oder AUA zu AUC verändert werden;
die Codons für Thr können von ACU oder ACA zu ACC oder ACG verändert werden;
das Codon für Asn kann von AAU zu AAC verändert werden;
das Codon für Lys kann von AAA zu AAG verändert werden;
die Codons für Val können von GUU oder GUA zu GUC oder GUG verändert werden;
das Codon für Asp kann von GAU zu GAC verändert werden;
das Codon für Glu kann von GAA zu GAG verändert werden.

Im Falle der Codons für Met (AUG) und Trp (UGG) besteht hingegen keine Möglichkeit der Sequenzmodifikation.

Die vorstehend aufgeführten Substitutionen können selbstverständlich einzeln aber auch in allen möglichen Kombinationen zur Erhöhung des G/C-Gehalts der modifizierten erfindungsgemäßen mRNA gegenüber der ursprünglichen Sequenz verwendet werden. So können beispielsweise alle in der ursprünglichen (Wildtyp-) Sequenz auftretenden Codons für Thr zu ACC (oder ACG) verändert werden. Vorzugsweise werden jedoch Kombinationen der vorstehenden Substitutionsmöglichkeiten genutzt, z.B.:
Substitution aller in der ursprünglichen Sequenz für Thr codierenden Codons zu ACC (oder ACG) und Substitution aller ursprünglich für Ser codierenden Codons zu UCC ( oder UCG oder AGC);
Substitution aller in der ursprünglichen Sequenz für Ile codierenden Codons zu AUC und Substitution aller ursprünglich für Lys codierenden Codons zu AAG und Substitution aller ursprünglich für Tyr codierenden Codons zu UAC;
Substitution aller in der ursprünglichen Sequenz für Val codierenden Codons zu GUC (oder GUG) und Substitution aller ursprünglich für Glu codierenden Codons zu GAG und Substitution aller ursprünglich für Ala codierenden Codons zu GCC (oder GCG) und Substitution aller ursprünglich für Arg codierenden Codons zu CGC (oder CGG);
Substitution aller in der ursprünglichen Sequenz für Val codierenden Codons zu GUC (oder GUG) und Substitution aller ursprünglich für Glu codierenden Codons zu GAG und Substitution aller ursprünglich für Ala codierenden Codons zu GCC (oder GCG) und Substitution aller ursprünglich für Gly codierenden Codons zu GGC (oder GGG) und Substituion aller ursprünglich für Asn codierenden Codons zu AAC;
Substitution aller in der ursprünglichen Sequenz für Val codierenden Codons zu GUC (oder GUG) und Substitution aller ursprünglich für Phe codierenden Codons zu UUC und Substitution aller ursprünglich für Cys codierenden Codons zu UGC und Substitution aller ursprünglich für Leu codierenden Codons zu CUG (oder CUC) und Substitution aller ursprünglich für Gln codierenden Codons zu CAG und Substitution aller ursprünglich für Pro codierenden Codons zu CCC (oder CCG); usw.

Vorzugsweise wird der G/C-Gehalt des für das Peptid bzw. Polypeptid codierenden Bereichs der modifizierten erfindungsgemäßen mRNA um mindestens 7%-Punkte mehr bevorzugt um mindestens 15%-Punkte, besonders bevorzugt um mindestens 20%-Punkte gegenüber dem G/C-Gehalt des codierten Bereichs der für das Polypeptid codierenden Wildtyp-mRNA erhöht.

Besonders bevorzugt ist es in diesem Zusammenhang, den G/C-Gehalt der modifizierten mRNA, insbesondere im für das mindestens eine Peptid bzw. Polypeptid codierenden Bereich, im Vergleich zur Wildtyp-Sequenz maximal zu erhöhen.

Weitere mögliche, nicht erfindungsgemäße, Modifikationen der in der vorliegenden Erfindung gekennzeichneten pharmazeutischen Zusammensetzung enthaltenen mRNA beruhen auf der Erkenntnis, daß die Translationseffizienz auch durch eine unterschiedliche Häufigkeit im Vorkommen von tRNAs in Zellen bestimmt wird. Sind daher in einer RNA-Sequenz vermehrt sogenannte "seltene" Codons vorhanden, so wird die entsprechende mRNA deutlich schlechter translatiert als in dem Fall, wenn für relativ "häufige" tRNAs codierende Codons vorhanden sind.

Somit kann in der modifizieren mRNA (weiche in der pharmazeutischen Zusammensetzung enthalten ist), der für das Peptid bzw. Polypeptid codierende Bereich gegenüber dem entsprechenden Bereich der Wildtyp-mRNA derart verändert, sein daß mindestens ein Codon der Wildtyp-Sequenz, das für eine in der Zelle relativ seltene tRNA codiert, gegen ein Codon ausgetauscht wird, das für eine in der Zelle relativ häufige tRNA codiert, welche die gleiche Aminosäure trägt wie die relativ seltene tRNA.

Durch diese nicht-erfindungsgemäße Modifikation werden die RNA-Sequenzen derart modifiziert, daß Codons eingefügt werden, für die häufig vorkommende tRNAs zur Verfügung stehen.

Welche tRNAs relativ häufig in der Zelle vorkommen und welche demgegenüber relativ selten sind, ist einem Fachmann bekannt; vgl. bspw. Akashi, Curr. Opin. Genet. Dev. 2001,11(6): 660-666.

Durch diese nicht-erfindungsgemäße Modifikation können alle Codons der Wildtyp-Sequenz, die für eine in der Zelle relativ seltene tRNA codieren, jeweils gegen ein Codon ausgetauscht werden, das für eine in der Zelle relativ häufige tRNA codiert, welche jeweils die gleiche Aminosäure trägt wie die relativ seltene tRNA.

Erfindungsgemäß besonders bevorzugt ist es, den erfindungsgemäß in der modifizierten mRNA erhöhten, insbesondere maximalen, sequenziellen G/C-Anteil mit den "häufigen" Codons zu verknüpfen, ohne die Aminosäuresequenz des durch den codierenden Bereich der mRNA codierten Peptids bzw. Polypeptids (ein oder mehrere) zu verändern. Diese bevorzugte Ausführungsform stellt eine besonders effizient translatierte und stabilisierte mRNA bspw. für die erfindungsgemäße pharmazeutische Zusammensetzung bereit.

In den Sequenzen eukaryotischer mRNAs gibt es destabilisierende Sequenzelemente (DSE), an welche Signalproteine binden und den enzymatischen Abbau der mRNA in vivo regulieren. Daher werden zur weiteren Stabilisierung der in der erfindungsgemäßen pharmazeutischen Zusammensetzung enthaltenen modifizierten mRNA gegebenenfalls im für das mindestens eine Peptid bzw. Polypeptid codierenden Bereich ein oder mehrere Veränderungen gegenüber dem entsprechenden Bereich der Wildtyp-mRNA vorgenommen, so daß keine destabilisierenden Sequenzelemente enthalten sind. Selbstverständlich ist es ebenfalls bevorzugt, gegebenenfalls in den nicht-translatierten Bereichen (3'- und/oder 5'-UTR) vorhandene DSE aus der erfindungsgemäßen mRNA zu eliminieren.

Derartige destabilisierende Sequenzen sind bspw. AU-reiche Sequenzen ("AURES"), die in 3'-UTR-Abschnitten zahlreicher instabiler mRNA vorkommen (Caput et al., Proc. Natl. Acad. Sci. USA 1986, 83: 1670 bis 1674). Die in der erfindungsgemäßen pharmazeutischen Zusammensetzung enthaltenen erfindungsgemäßen RNA-Molekule sind daher vorzugsweise derart genüber der Wildtyp-mRNA verändert, daß sie keine derartigen destabilisierenden Sequenzen aufweisen. Dies gilt auch für solche Sequenzmotive, die von möglichen Endonucleasen erkannt werden, bspw. die Sequenz GAACAAG, die im 3' UTR-Segment des für den Transferin-Rezeptor codierenden Gens enthalten ist (Binder et al., EMBO J. 1994, 13: 1969 bis 1980). Auch diese Sequenzmotive werden bevorzugt in der modifizierten mRNA der erfindungsgemäßen pharmazeutischen Zusammensetzung eliminiert.

Einem Fachmann sind verschiedene Verfahren geläufig, die zur Substitution von Codons in der erfindungsgemäßen modifizierten mRNA geeignet sind. Im Falle kürzerer codierender Bereiche (die für biologisch wirksame oder antigene Peptide codieren) kann bspw. die gesamte mRNA chemisch unter Verwendung von Standardtechniken synthetisiert werden.

Bevorzugt werden allerdings Basensubstitutionen unter Verwendung einer DNA-Matrize zur Herstellung der modifizierten mRNA mit Hilfe von Techniken der gängigen zielgerichteten Mutagenese eingeführt; Maniatis et al., Molecular Cloning: A Laboratory Manual, Cold Spring Harbor Laboratory Press, 3. Aufl., Cold Spring Harbor, NY, 2001.

Bei diesem Verfahren wird zur Herstellung der erfindungsgemäßen mRNA daher ein entsprechendes DNA-Molekül *in vitro* transkribiert. Diese DNA-Matrize besitzt einen geeigneten Promotor, bspw. einen T7 -oder SP6-Promotor, für die *in vitro* Transkription, dem die gewünschte Nucleotidsequenz für die herzustellende mRNA und ein Termisationssignal für die in *in vitro* Transkribtion folgen. Das DNA-Molekül, das die Matrize des herzustellenden RNA-Konstrukts bildet, kann durch fermentative Vermehrung und anschließende Isolierung als Teil eines in Bakterien replizierbaren Plasmids hergestellt werden. Als für die vorliegende Erfindung geeignete Plasmide können bspw. die Plasmide pT7Ts (GenBank-Zugriffsnummer U26404; Lai et al., Development 1995, 121: 2349 bis 2360), pGEM^{®}-Reihe, bspw. pGEM^{®}-1 (GenBank-Zugriffsnummer X65300; von Promega) und pSP64 (GenBank-Zugriffsnummer X65327) genannt werden; vgl. auch Mezei und Storts, Purification of PCR Products, in: Griffin und Griffin (Hrsg), PCR Technology: Current Innovation, CRC Press, Boca Raton, FL, 2001.

Es kann so unter Verwendung kurzer synthetischer DNA-Oligonucleotide, die an den entstehenden Schnittstellen kurze einzelsträngige Übergänge aufweisen, oder durch chemische Synthese hergestellte Gene die gewünschte Nucleotidsequenz nach einem Fachmann geläufigen molekularbiologischen Methoden in ein geeignetes Plasmid cloniert werden (vgl. Maniatis et al., s.o.). Das DNA-Molekül wird dann aus dem Plasmid, in welchem es in einfacher oder mehrfacher Kopie vorliegen kann, durch Verdauung mit Restrildionsendonukleasen ausgeschnitten.

Die erfindungsgemäße modifizierte mRNA, welche in der erfindungsgemäßen pharmazeutischen Zusammensetzung enthalten ist, kann darüber hinaus eine 5'-Cap-Struktur (ein modifiziertes Guanosin-Nucleotid) aufweisen. Als Beispiele von Cap-Strukturen können m7G(5')ppp (5'(A,G(5')ppp(5')A und G(5')ppp(S')G genannt werden.

Gemäß einer weiteren bevorzugten Ausführungsform der vorliegenden Erfindung enthält die modifizierte erfindungsgemäße mRNA einen PolyA-Schwanz von mindestens 50 Nucleotiden, vorzugsweise mindestens 70 Nuclotiden, mehr bevorzugt mindestens 100 Nucleotiden, besonders bevorzugt mindestens 200 Nucleotiden.

Für eine effiziente Translation der erfindungsgemäßen mRNA ist weiterhin eine wirksame Bindung der Ribosomen an die Ribosomen-Bindungsstelle (Kozak-Sequenz: GCCGCCACCAUGG. das AUG bildet das Startcodon) erforderlich. Diesbezüglich ist festgestellt worden, daß ein erhöhter A/U-Gehalt um diese Stelle herum eine effizientere Ribosomen-Bindung an die mRNA ermöglicht

Des weiteren ist es möglich, in die modifizierte erfindungsgemäße mRNA eine oder mehrere sog. IRES (engl. "internal ribosomal entry side) einzufügen. Eine IRES kann so als alleinige Ribosomen-Bindungsstelle fungieren, sie kann jedoch auch zur Bereitstellung einer mRNA dienen, die mehrere Peptide bzw. Polypeptide codiert, die unabhängig voneinander durch die Ribosomen translatiert werden sollen ("multicistronische mRNA'). Beispiele erfindungsgemäß verwendbarer IRES-Sequenzen sind diejenigen aus Picornaviren (z.B. FMDV), Pestviren (CFFV), Polioviren (PV), Enzephalo-Myocarditis-Viren (ECMV), Maul-und-Klauenseuche-Viren (FMDV), Hepatitis-C-Viren (HCV), Klassisches-Schweinefieber-Viren (CSFV), Murines-Leukoma-Virus (MLV), Simean-Immundefizienz-Viren (SIV) oder Cricket-Paralysis-Viren (CrPV).

Gemäß einer weiteren bevorzugten Ausführungsform der vorliegenden Erfindung weist die modifizierte erfindungsgemäße mRNA in den 5'-und/oder 3'-nicht translatierten Bereichen Stabilisierungssequenzen auf, die befähigt sind, die Halbwertszeit der mRNA im Cytosol zu erhöhen.

Diese Stabilisierungssequenzen können eine 100%ige Sequenzhomologie zu natürlich vorkommenden Sequenzen, die in Viren, Bakterien und Eukaryoten auftreten, aufweisen, können aber auch teilweise oder vollständig synthetischer Natur sein. Als Beispiel für stabilisierende Sequenzen, die in der vorliegenden Erfindung verwendbar sind, können die nicht-translatierten Sequenzen (UTR) des β-Globingens, bspw. von *Homo sapiens* oder *Xenopus laevis,* genannt werden. Ein anderes Beispiel einer Stabilisierungssequenz weist die allgemeine Formel (CJU)CCAN_{R}CCC(UJA)PyₓUC(CJU)CC auf, die im 3'UTR der sehr stabilen mRNA enthalten ist, die für α-Globin, α-(I)-Collagen 15-Lipoxygenase oder für Tyrosin-Hydroxylase codiert (vgl. Holcik et al., Proc. Natl. Acad. Sci. USA 1997, 94: 2410 bis 2414). Selbstverständlich können derartige Stabilisienmgssequenzen einzeln oder in Kombination miteinander als auch in Kombination mit anderen, einem Fachmann bekannten Stabilisierungssequenzen verwendet werden.

Zur weiteren Stabilisierung der modifizierten erfindungsgemäßen mRNA ist es außerdem bevorzugt, daß diese mindestens ein Analoges natürlich vorkommender Nucleotide aufweist Dies beruht auf der Tatsache, daß die in den Zellen vorkommenden RNA-abbauenden Enzyme als Substrat vorzugsweise natürlich vorkommende Nucleotide erkennen. Durch Einfügen von Nucleotidanaloga kann daher der RNA-Abbau erschwert werden, wobei die Auswirkung auf die Translationseffizienz bei Einfügen von diesen Analoga, insbesondere in den codierenden Bereich der mRNA, einen positiven oder negativen Effekt auf die Translationseftizienz haben kann.

In einer keineswegs abschließenden Aufzählung können als Beispiele erfindungsgemäß verwendbarer Nucleotidanaloga Phosphoramidate, Phosphorthioate, Peptidnucleotide, Methylphosphonate, 7-Deazaguaonsin, 5-Methylcytosin und Inosin genannt werden. Die Herstellung derartiger Analoga sind einem Fachmann bspw. aus den US-Patenten 4,373,071, US 4,401,796, US 4,415,732, US 4,458,066, US 4,500,707, US 4,668,777, US 4,973,679, US 5,047,524, US 5,132,418, US 5,153,319, US 5,262,530 und 5,700,642 bekannt Erfindungsgemäß können derartige Analoga in nicht-translatierten und translatierten Bereichen der modifizierten erfindungsgemäßen mRNA vorkommen.

Des weiteren kann der wirksame Transfer der modifizierten erfindungsgemäßen mRNA in die zu behandelnden Zellen bzw. den zu behandelnden Organismus dadurch verbessert werden, daß die modifizierte mRNA mit einem kationischen Peptid oder Protein assoziiert oder daran gebunden ist. Insbesondere ist dabei die Verwendung von Protamin als polykationischem, Nucleinsäure-bindenden Protein besonders wirksam. Des weiteren ist die Verwendung anderer kationischer Peptide oder. Proteine, wie Poly-L-Lysin oder Histonen, ebenfalls möglich. Diese Vorgehensweise zur Stabilisierung der modifizierten erfindungsgemäßen mRNA ist in EP-A-1083232 beschrieben.

Ein möglicher Anwendungsbereich der vorliegenden Erfindung ist die Vakzinierung, d.h. die Verwendung der modifizerten erfindungsgemässen mRNA zur Impfung bzw. die Verwendung der pharmazeutischen Zusamensetzung als Impfstoff bzw. die erfindungsgemässe Verwendüng der modifizierten erfindungsgemässen mRNA zur Herstellung der pharmazeutishen Zusammesetzung zur Impfung. Die Vakzinierung beruht auf der Einbringung eines (Tumor-) Antigens, im vorliegenden Fall der genetischen Information für das Kantigen in Form der für das Antigen codierenden modifizierten mRNA, in den Organismus, insbesondere in die Zelle. Die in der pharmazeutischen Zusammensetzung enthaltene modifiziererte erfindungsgemässe mRNA wird in das Antigen translatiert, d.h. das von der modifizierten mRNA codierte Polypeptid bzw. antigene Peptid wird exprimiert, wodurch eine gegen dieses Polypeptid bzw. antigene Peptid gerichtete Immunantwort stimuliert wird. Im Fall der Verwendung als genetischen Vakzine zur Behandlung von Krebs wird die Immunantwort durch Einbringung der genetischen Information für Tumorantigene, insbesondere Proteine, die ausschließlich auf Krebszellen exprimiert werden, erreicht, in dem eine erfindungsgemäße pharmazeutischen Zusammensetzung verabreicht wird, die eine für ein derartiges Krebsantigen codierende erfindungsgemässe mRNA enthält. Dadurch wird das oder die Krebsantigen(e) im Organismus exprimiert, wodurch eine Immunantwort hervorgerufen wird, die wirksam gegen die Krebszellen gerichtet ist.

In ihrer Verwendung als Vakzine kommt die erfindungsgemäße pharmazeutische Zusammensetzung insbesondere zur Behandlung von Krebserkrankungen (wobei die modifizierte mRNA für ein tumorspezifisches Oberflächenantigen (TSSA) codiert), bspw. zur Behandlung von malignem Melanom, Kolon-Karzinom, Lymphomen Sarkomen, kleinzelligem Lungenkarzinom, Blastomen usw., in Betracht. Spezifische Beispiele von Tumorantigenen sind u.a. 707-AP, AFP, ART-4, BAGE, β-Cateninlm, Bcr-abl, CAMEL, CAP-1, CASP.8, CDC27/m, CDK4/m, CEA, CT, Cyp-B, DAM, ELF2M, ETV6-AMLl, G250, GAGE, GnT-V, Gp100, HAGE, HER-2/neu, HLA-A*0201-R170I, HPV-E7, HSP70-2M, HAST-2, hTERT (oder hTRT), iCE, KIAA0205, LAGE, LDLR/FUr, MAGE, MART-1/Melan-A, MC 1 R, Myosin/m, MUC1, MUM-1, -2, 3, NA88-A, NY-ESO-1, p190 minor ber-ab1, Pm1/RARα, PRAME, PSA, PSM, RAGE, RU1 oder RU2, SAGE, SART-1 oder SART-3, TEL/AML1, TPI/m, TRP-1, TRP-2, TRP-2/INT2 und WT1. Des weiteren werden erfindungsgemäß bevorzugt für Polypeptide codierende mRNAs eingesetzt, wobei es sich bei den Polypeptiden um Polyepitope, bspw. der vorstehend genannten Antigene, insbesondere Oberflächenantigene von Tumorzellen vorzugsweise sekretierter Proteinformen, handelt.

Darüber hinaus kann die erfindungsgemäß modifizierte mRNA neben dem antigenen Peptid bzw. Polypeptid auch mindestens einen weiteren funktionalen Abschnitt enthalten, der bspw. für ein die Immunantwort förderndes Cytokin (Monokin, Lymphokin, Interleukin oder Chemokin, wie IL-I, IL-2, IL.-3, IL-4, IL-5, IL-6, IL-7, IL-8, IL-9, IL-10, IL-12, INF-α, LNF-γ, GM-CFS, LT-α oder Wachstumsfaktoren, wie hGH, codiert.

Des weiteren kann zur Erhöhung der Immunogenizität die erfindungsgemäße pharmazeutische Zusammensetzung ein oder mehrere Adjuvanzien enthalten. Unter "adjuvans" ist dabei jede chemische oder biologische Verbindung zu verstehen, die eine spezifische Immunantwort begünstigt. In Abhängigkeit der verschiedenen Arten von Adjuvanzien können diesbezüglich verschiedene Mechanismen in Betracht kommen. Bspw. bilden Verbindungen, die eine Endocytose der in der pharmazeutischen Zusammensetzung enthaltenen modizierten erfindungsgemäßen mRNA durch dendritische Zellen (DC) fördern, eine erste Klasse von verwendbaren Adjuvanzien. Andere Verbindungen, welche die Reifung der DC erlauben, bspw. Lipopolysaccharide, TNF-α oder CD40-Ligand, sind eine weitere Klasse geeigneter Adjuvanzien. Allgemein kann jedes das Immunsystem beeinflussende Agens von der Art eines "Gefahrsignals" (LPS, GP96, Oligonucleotide mit dem CpG-Motiv) oder Cytokine, wie GM-CFS, als Adjuvans verwendet werden welche es erlauben, eine Immunantwort gegen ein Antigen, das durch die modifizierte erfindungsgemäße mRNA codiert wird, zu erhöhen und/oder gerichtet zu beeinflussen. Insbesondere sind dabei die vorstehend genannten Cytokine bevorzugt. Weitere bekannte Adjuvanzien sind Aluminiumhydroxid, das Freud'sche Adjuvans sowie die vorstehend genannten stabilisierenden kationischen Peptide bzw. Polypeptide, wie Protamin. Des weiteren sind Lipopeptide, wie Pam3Cys, ebenfalls besonders geeignet, um als Adjuvanzien in der pharmazeutischen Zusammmensetzung der vorliegenden Erfindung eingesetzt zu werden; vgl. Derer et al, Nature 1989, 342: 561-564.

Die erfindungsgemäße pharmazeutische Zusammensetzung enthält neben der modifizierten erfindungsgemäßen mRNA einen pharmazeutisch verträglichen Träger und/oder ein pharmazeutisch verträgliches Vehikel. Entsprechende Wege zur geeigneten Formulierung und Herstellung der erfindungsgemäßen pharmazeutischen Zusammensetzung sind bei "Remington's Pharmaceutical Sciences" (Mack Pub. Co., Easton, PA, 1980) offenbart. Für die parenterale Verabreichung kommen als Trägerstoffe bspw. steriles Wasser, sterile Kochsalzlösungen, Potyalkylenglykole, hydrogenierte Naphthalen und insbesondere biokompatible Lactidpolymere, Lactid/Glycolidcopolymer oder Polyoxyethylen-/Polyoxypropylencopolymere in Betracht. Erfindungsgemäße Zusammensetzungen können Fullsubstanzen oder Substanzen, wie Lactose, Mannitol, Substanzen zur kovalenten Anknüpfung von Polymeren, wie z.B. Polyethylenglykol an erfindungsgemäße Inhibitoren, Komplexierung mit Metaltionen oder Einschluß von Materialien in oder auf besondere Präparationen von Polymerverbindung, wie z.B. Polylactat, Polyglykolsäure, Hydrogel oder auf Liposomen, Mikroemulsion, Micellen, unilamellare oder multilamellare Vesikel, Erythrozyten-Fragmente oder Sphäroplasten, enthalten. Die jeweiligen Ausführungsformen der Zusammensetzungen werden abhängig vom physikalische Verhalten, beispielsweise in Hinblick auf die Löslichkeit, die Stabilität, Bioverfügbarkeit oder Abbaubarkeit gewählt. Kontrollierte oder konstante Freisetzung der erfindungsgemäßen Wukstoffkomponente in der Zusammensetzung schließt Formulierungen auf der Basis lipophiler Depots ein (z.B. Fettsäuren, Wachse oder Öle). Im Rahmen der vorliegenden Erfindung werden auch Beschichtungen erfindungsgemäßer Substanzen oder Zusammensetzungen, enthaltend solche Substanzen, nämlich Beschichtungen mit Polymeren offenbart (z.B. Poloxamere oder Poloxamine). Weiterhin können erfindungsgemäßen Substanzen bzw. Zusammensetzungen protektive Beschichtungen, z.B. Proteaseinhibitoren oder Permeabilitätsverstärker, aufweisen. Bevorzugte Träger sind typischerweise wässrige Trägermaterialien, wobei Wasser zur Injektion (WFI) oder Wasser, gepuffert mit Phosphat, Citrat oder Acetat usw. verwendet wird, und der pH typischerweise auf 5,0 bis 8,0, vorzugsweise 6,0 bis 7,0, eingestellt wird. Der Träger bzw. das Vehikel wird zusätzlich vorzugsweise Salzbestandteile enthalten, z.B. Natriumchlorid, Kaliumchlorid oder andere Komponenten, welche die Lösung bspw. isotonisch machen. Weiterhin kann der Träger bzw. das Vehikel neben den vorstehend genannten Bestandteilen zusätzliche Komponenten, wie humanes Serumalbumin (HSA), Polysorbat 80, Zucker oder Aminosäuren, enthalten.

Die Art und Weise der Verabreichung und die Dosierung der erfindungsgemäßen pharmazeutischen Zusammensetzung hängen von der zu behandelnden Erkrankung und deren Fortschriftsstadium, wie auch dem Körpergewicht, dem Alter und dem Geschlecht des Patienten ab.

Die Konzentration der modifizierten erfindungsgemäßen mRNA in derartigen Formulierungen kann daher innerhalb eines weiten Bereichs von 1 µg bis 100 mg/ml variieren. Die erfindungsgemäße pharmazeutische Zusammensetzung wird vorzugsweise parenteral, bspw. intravenös, intraarteriell, subkutan, intramuskulär, dem Patienten verabreicht Ebenso ist es möglich, die pharmazeutische Zusammensetzung topisch oder oral zu verabreichen

Somit kann bspw. auch ein Verfahren zur Behandlung der vorstehend genannten Krankheiten bzw. ein Impfverfahren zur Prävention der vorstehend genannten Erkrankungen eingesetzt werden, welches das Verabreichen der erfindungsgemäßen pharmazeutischen Zusammensetzung an einen Patienten, insbesondere einen Menschen, umfasst.

Darüber hinau kann ebenfalls ein Verfahren ein gesetzt werden das der Ermittlung der modifizierten Sequenz der in der erfindungsgemäßen pharmazeutischen Zusammensetzung enthaltenen mRNA dient. Dabei kann bspw. die Adaptierung der RNA-Sequenzen mit zwei unterschiedlichen Optimierungszielen durchgeführt werden. Zum einen mit größtmöglichen G/C-Cehalt, zum anderen ggf. unter bestmöglicher Berücksichtigung der Häufigkeit der tRNAs gemäß Codon Usage. Im ersten Schritt des Verfahrens wird eine virtuelle Translation einer beliebigen RNA(oder DNA)-Sequenz durchgeführt, um die entsprechende Aminosäuresequenz zu generieren. Ausgehend von der Aminosäuresequenz wird eine virtuelle reverse Translation durchgeführt, die aufgrund des degenerierten genetischen Codes Wahlmöglicdzkeiten für die entsprechenden Codons liefert. Je nach geforderter Optimiorung bzw. Modifizierung werden zur Auswahl der geeigneten Codons entsprechende Selektionslisten und Optimierungsalgorithmen verwendet. Die Ausführung der Algorithmen erfolgt typischerweise mit Hilfe einer geeigneten Software auf einem Computer. So wird die optimierte mRNA-Sequenz erstellt und kann bspw. mit Hilfe einer entsprechenden Anzeigevorrichtung ausgegeben und mit der usrpünglichen (Wildtyp-)Soquenz verglichen werden. Das gleiche gilt auch für die Häufigkeit der einzelnen Nucleotide. Die Änderungen gegenüber der ursprünglichen Nucleotidsequenz werden dabei vorzugsweise hervorgehoben. Weiter werden gemäß einer bevorzugten Ausführungsform in der Natur bekannte stabile Sequenzen eingelesen, welche die Grundlage für eine gemäß natürlichen Sequenzmotiven stabilisierte RNA bieten können. Es kann ebenfalls eine Sekundätstrukturanalyse vorgesehen werden, die anhand von Strukturberechnungen stabilisierende und iostabilisiereade Eigenschaften bzw. Bereiche der RNA analysieren kann.

Die Figuren zeigen:

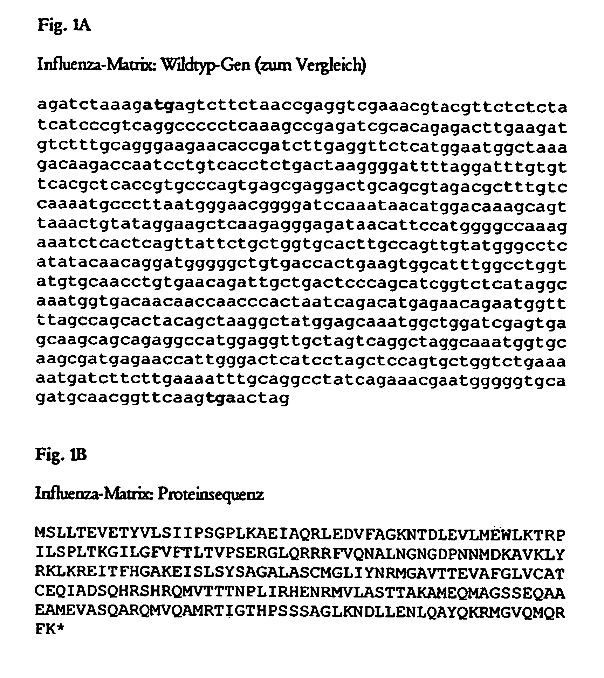

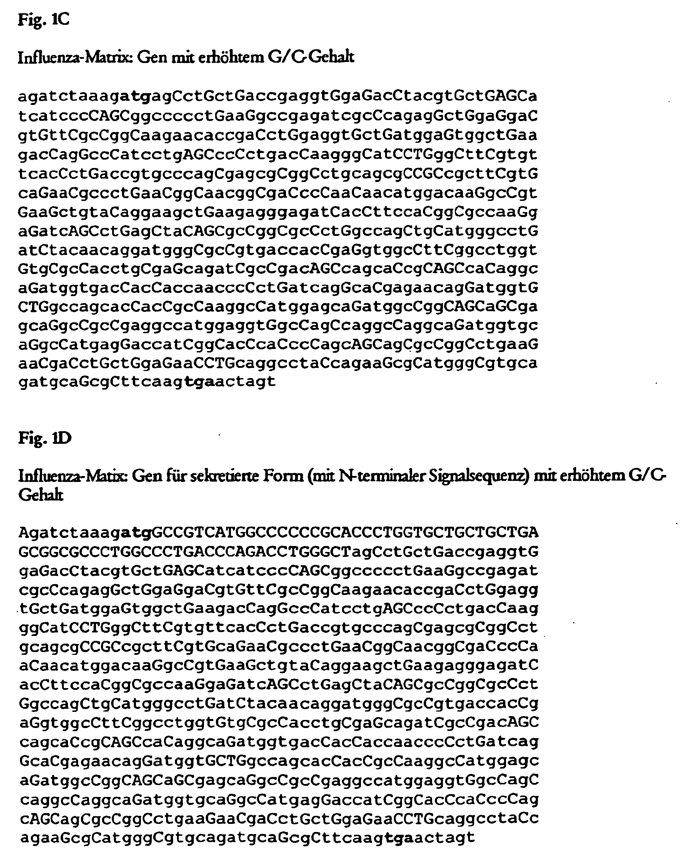

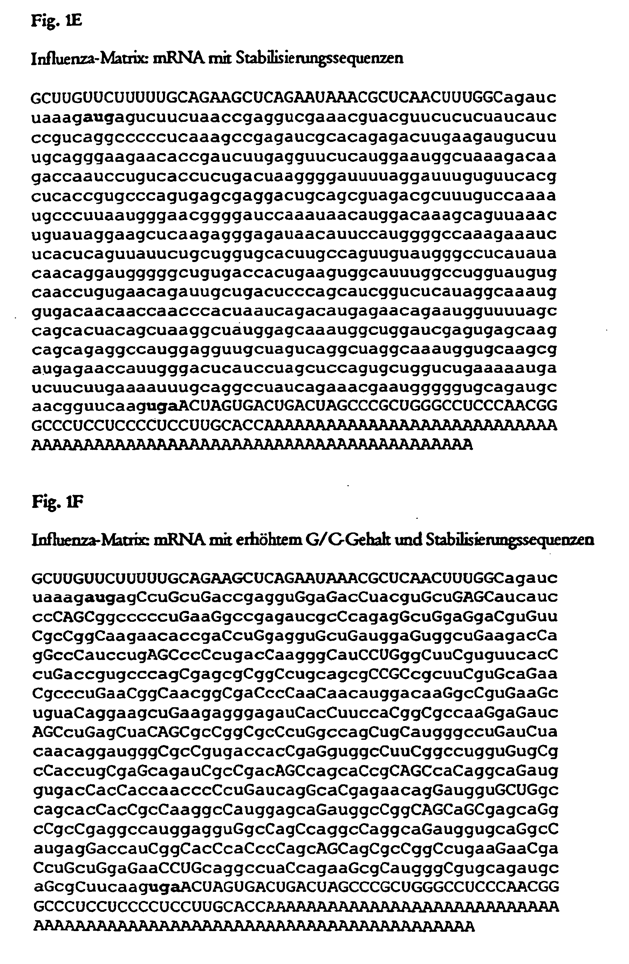

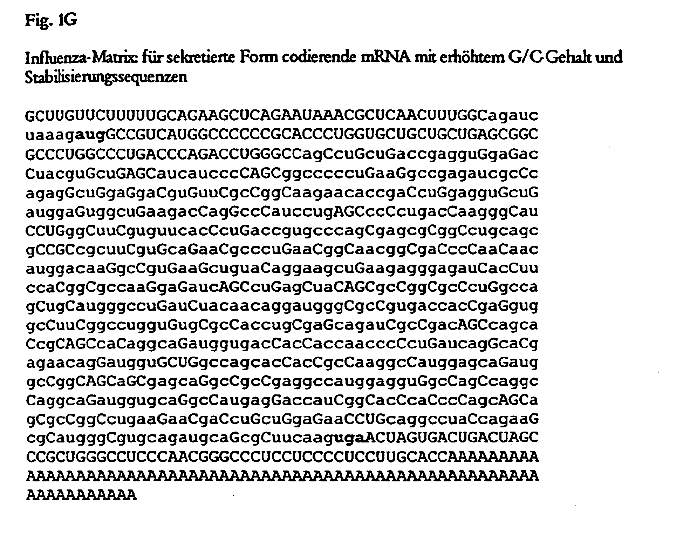

Fig. 1 zeigt Wildtyp- und nicht-erfindungsgemäße modifizierte Sequenzen für das Influenza-Matrix-Protein. Fig. 1A zeigt das Wildtyp-Gen und Fig. 1B zeigt die davon abgeleitete Aminosäuresequenz (Ein-Buchstaben-Code). Fig. 1C zeigt eine für das Influenza-Matrix-Protein codierende Gen-Sequenz, deren G/C-Gehalt gegenüber der Wildtyp-Sequenz erhöht ist. Fig. 1D zeigt die Sequenz eines Gens, das für eine sekretierte Form des Influenza-Matrix-Proteins codiert (einschließlich einer N-terminalen Signalsequenz), wobei der G/C-Gehalt der Sequenz gegenüber der Wildtyp-Sequenz erhöht ist. Fig. 1E zeigt eine für das Influenza-Matrix-Protein codierende mRNA, die im Vergleich zur Wildtyp-mRNA stabilisierende Sequenzen enthält. Fig. 1F zeigt eine für das Influenza-Matrix-Protein codierende nicht-erfindungsgemäße mRNA, die neben stabilisierenden Sequenzen einen erhöhten G/C-Gehalt aufweist.
Fig. 1G zeigt ebenfalls eine modifiziertze nicht-erfindungsgemäße mRNA, die für die sekretierte Form des Influenza-Matrix-Proteins codiert und im Vergleich zum Wildtyp stabilisierende Sequenzen und einen erhöhten GC-Gehalt aufweist. In Fig. 1A und Fig. 1C bis 1G sind die Start- und Stop-Codons fett hervorgehoben. Gegenüber der Wildtyp-Sequenz von Fig. 1A veränderte Nucleotide sind in den Figuren 1C bis 1G in Großbuchstaben dargestellt.

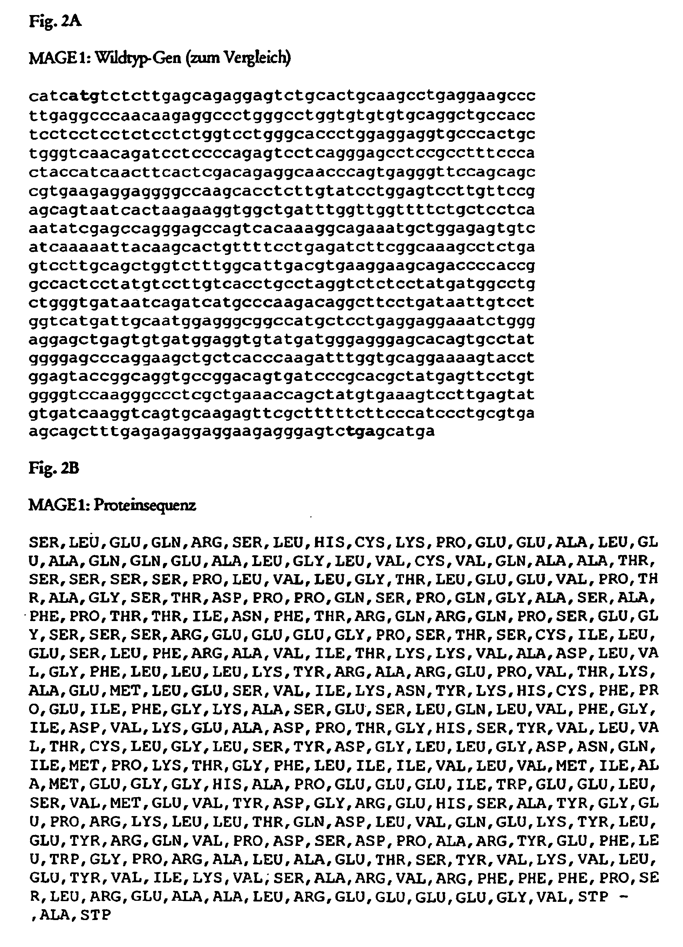

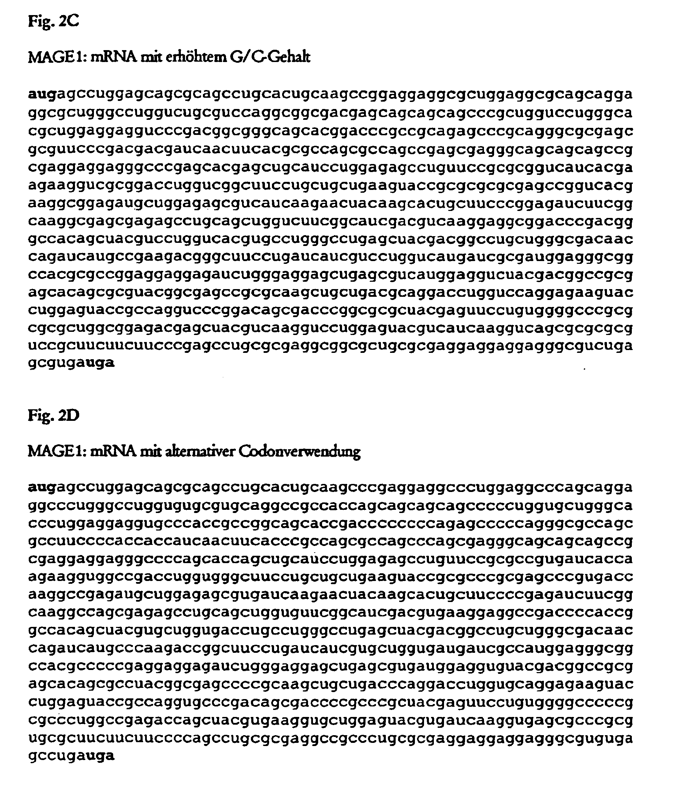

Fig. 2 zeigt Wildtyp- und erfindungsgemäß modifizierte Sequenzen, die für das Tumorantigen MAGE1 codieren.
Fig. 2A zeigt die Sequenz des Wildtyp-Gens und Fig. 2B die davon abgeleitete Aminosäuresequenz (Drei-Buchstaben-Code). Fig. 2C zeigt eine für MAGE1 codierende modifizierte mRNA, deren G/C-Gehalt gegenüber dem Wildtyp erhöht ist. Fig. 2D zeigt die Sequenz einer für MAGE1 codierenden modifizierten mRNA, bei der die Codon-Verwendung bezüglich der Codierung möglichst häufig in der Zelle vorkommender tRNA optimiert wurde. Start- und Stop-Codons sind jeweils fett gekennzeichnet.

Die folgenden Beispiele erläutern die vorliegende Erfindung näher, ohne sie einzuschränken.

### Beispiel 1

Als beispielhafte Ausführungsform des Verfahrens zu Ermittlung der Sequenz einer erfindungsgemäß modifizierten mRNA wurde ein Computerprogramm erstellt, das mit Hilfe des genetischen Codes bzw. dessen degenerativer Natur die Nucleotid-Sequenz einer beliebigen mRNA derart modifiziert, daß sich ein maximaler G/C-Gehalt in Verbindung mit der Verwendung von Codons, die für möglichst häufig in der Zelle vorkommende tRNAs codieren, ergibt, wobei die durch die modifizierte mRNA codierte Aminosäure-Sequenz gegenüber der nicht-modifizierten Sequenz identisch ist. Alternativ kann auch nur der G/C-Gehalt oder nur die Codonverwendung gegenüber der ursprünglichen Sequenz modifiziert werden.

Der Quellcode in Visual Basic 6.0 (eingesetzte Entwicklungsumgebung. Microsoft Visual Studio Enterprise 6.0 mit Servicepack 3) ist im Anhang angegeben.

### Beispiel 2

Mit Hilfe des Computerprogramms von Beispiel 1 wurde ein RNA-Konstrukt mit einer hinsichtlich Stabilisierung und Translationseffizienz optimierten Sequenz des lac-Z-Gens aus *E.coli* erstellt. Es konnte so ein G/C-Gehalt von 69% (im Vergleich zur Wildtyp-Sequenz von 51%; vgl. Kalnins et al., EMBO J. 1983, 2(4): 593-597) erzielt werden. Durch die Synthese überlappender Oligonucleotide, welche die modifizierte Sequenz aufweisen, wurde die optimierte Sequenz gemäß im Stand der Technik bekannter Verfahren hergestellt. Die endständigen Oligonucleotide wiesen die folgenden Restriktionsscbnittstellen auf-. Am 5'-Ende eine EcoRV- sowie am 3'-Ende eine BglII-Schnittstelle. Über Verdau mit *Eco*RV/*Bgl*II wurde die modifizierte lacZ-Sequenz in das Plasmid pT7Ts. (GenBank-Zugriffsnummer U 26404; vgl. Lai et al., s.o.). p17Ts enthält als nichttranslatierte Regionen Sequenzen aus dem β-Globingen von *Xenopus levis* jeweils an 5' und 3'. Das Plasmid wurde vor dem Einfügen der modifizierten lacZ-Sequenz mit den genannten Restriktionsenzymen geschnitten.

Das pT7Ts-lac-Z-Konshukt wurde in Bakterien vermehrt und durch Phenol-Chloroform-Extraktion gereinigt. 2 µg des Konstrukts wurden *in vitro* mittels dem einem Fachmann bekannten Verfahren transkribiert, wodurch die modifizierte mRNA hergestellt wurde.

### Beispiel 3

Mit Hilfe des Computerprogramms von Beispiel 1 wurde das Gen für das Influenza-Matrix-Protein (Wildtyp-Sequenz vgl. Fig. 1A, abgeleitete Aminosäuresequenz Fig. 1B) optimiert. Dadurch wurde die in Fig. 1C dargestellte G/C-reiche Sequenzvariante erstellt. Ebenfalls wurde eine für die sekretierte Form des Muem-Matrix-Proteins codierende G/C-reiche Sequenz, die für eine N-terminale Signalsequenz codiert, ermittelt (vgl. Fig. 1D). Die sekretierte Form des lnfluenza-Matrix-Proteins hat den Vorteil, daß sie eine im Vergleich zur nicht-sekretierten Form erhöhte Immunogenizität aufweist.

Ausgehend von den optimierten Sequenzen wurden entsprechende nicht-erfindungsgemäße mRNA-Moleküle entworfen. Die hinsichtlich G/C-Gehalt und Codonverwendung optimierte mRNA für das Influenza-Matrix-Protein wurde zusätzlich mit stabilisierenden Sequenzen im 5'- und 3'-Bereich (die Stabilisierungssequenzen stammen aus den 5'- bzw. 3'-UTRs des β-Globin-mRNA von *Xenupus laevis;* vgl. pTTTs-Vektor in Lai et al., s.o.) ausgestattet (vgl. Fig. 1E und 1F). Desgleichen wurde auch die für die sekretierte Form des Influenza-Matrix-Proteins codierende mRNA im translatierten Bereich sequenzoptimiert und mit den genannten stabilisierenden Sequenzen versehen (vgl. Fig. 1G).

### Beispiel 4

Mit Hilfe des Computerprogramms von Beispiel 1 wurde die für das Tumorantigen MA-GEI codierende mRNA modifiziert. Dadurch wurde die in Fig. 2C gezeigte Sequenz ermittelt, die im Vergleich zur Wildtyp-Sequenz (275 G, 244 G) einen um 24% höheren G/C-Gehalt aufweist (351 G, 291 C). Des weiteren wurde die Wildtyp-Sequenz durch alternative Codonverwendung hinsichtlich der Translationseffizienz aufgrund der Codierung von in der Zelle häufiger vorkommenden tRNAs verbessert (vgl. Fig. 2D). Durch die alternative Codonverwendung wurde der G/C-Gehalt ebenfalls um 24% erhöht.

### Anhang: Quelltext eines beispielhafen Computerprogramms

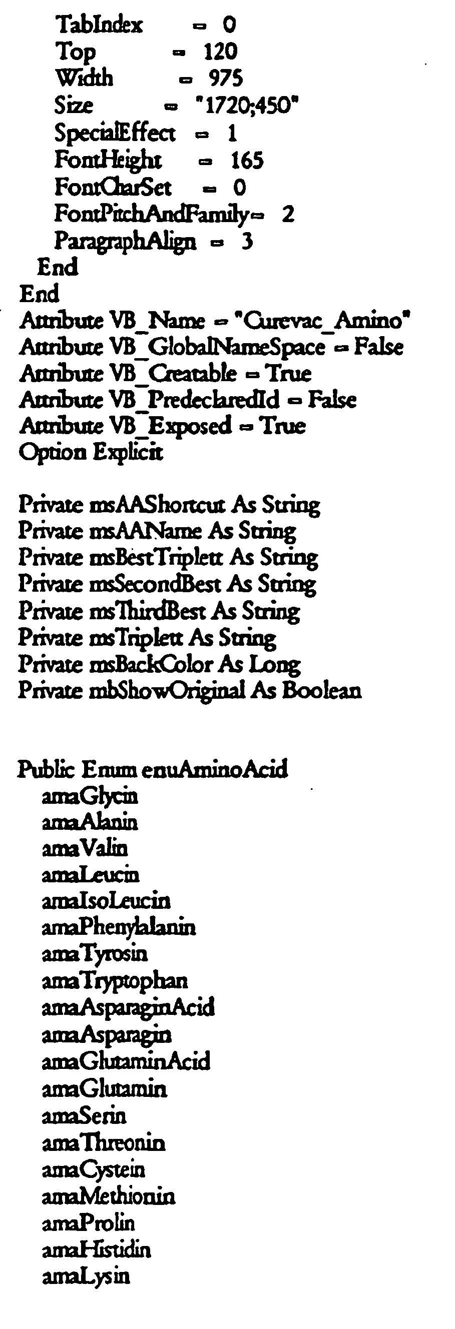

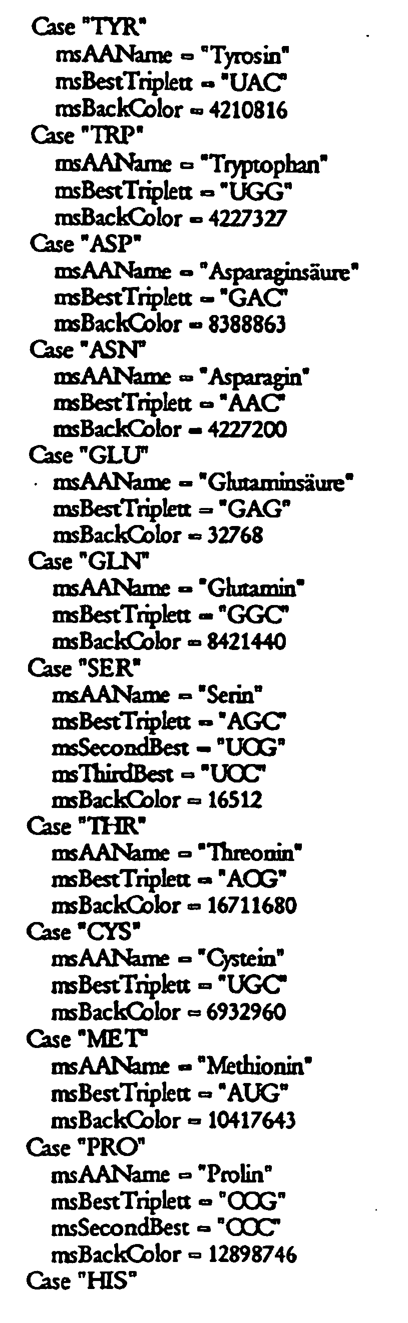

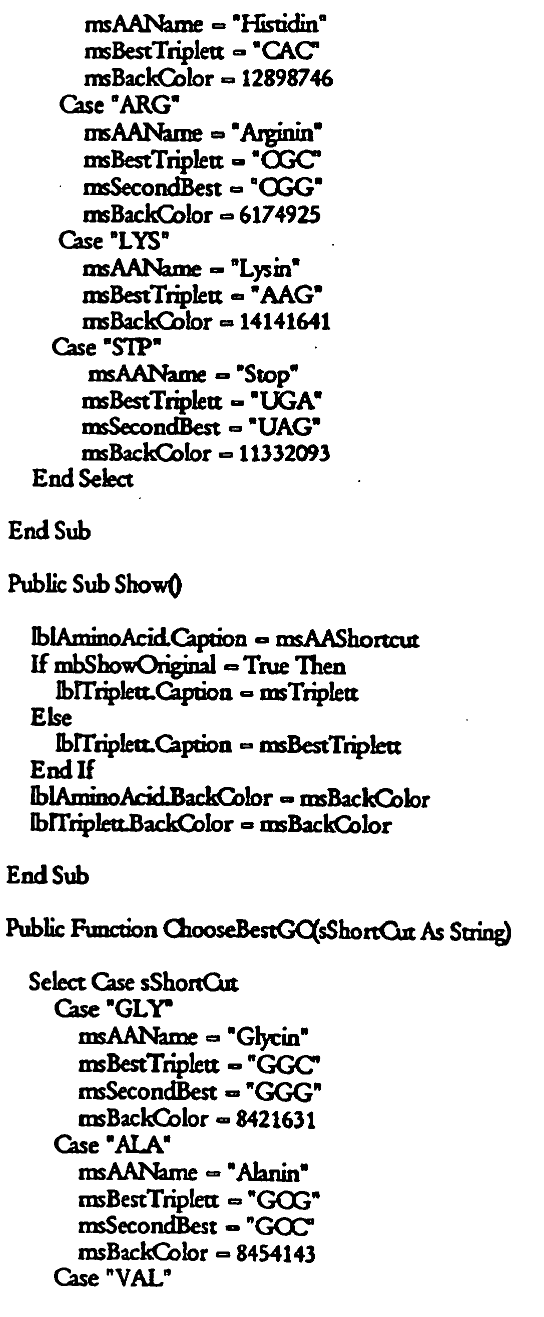

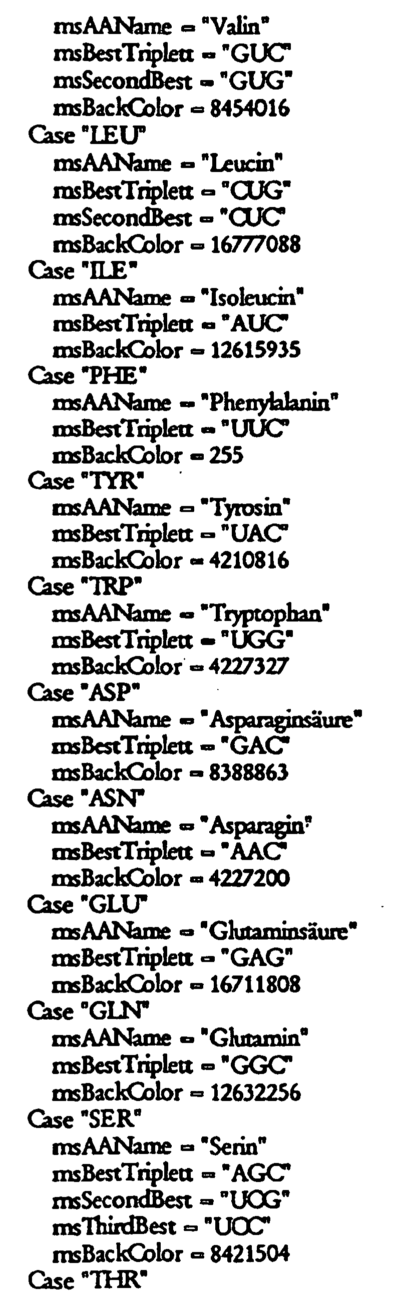

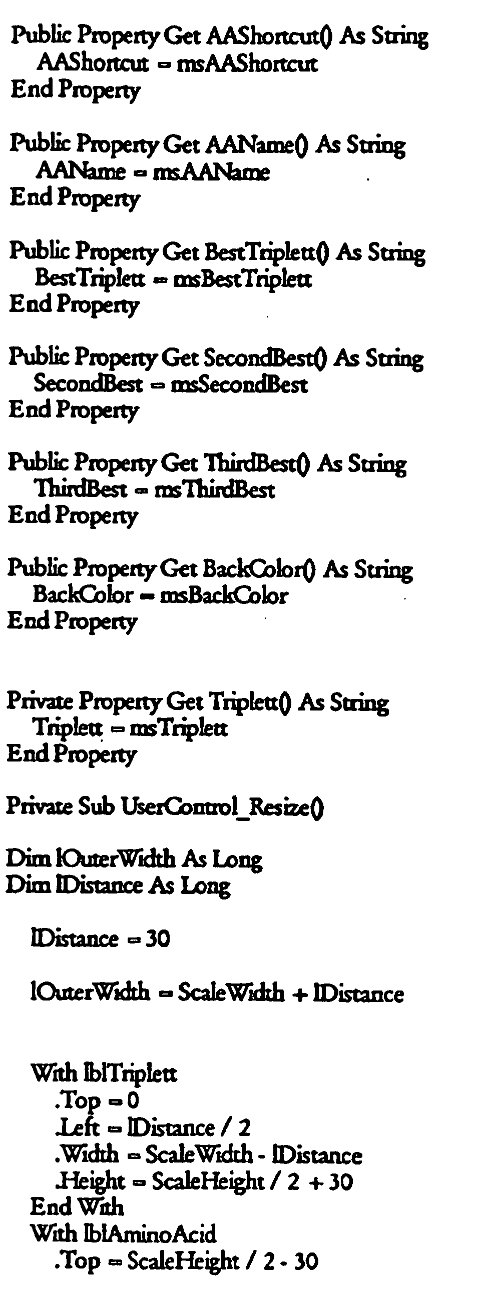

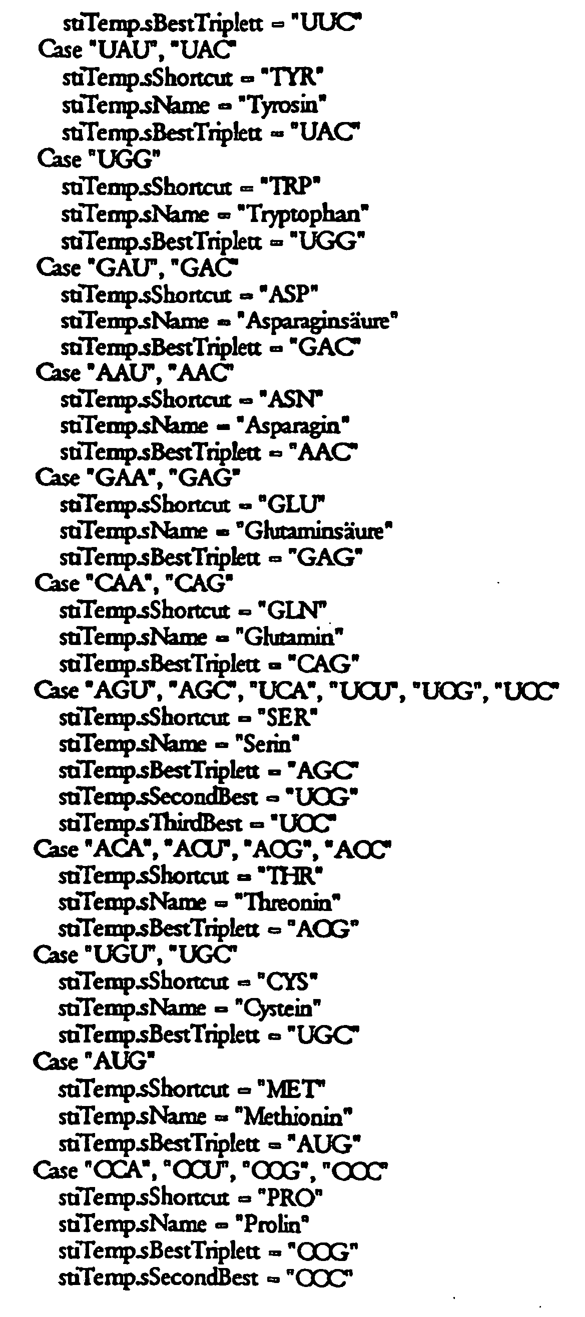

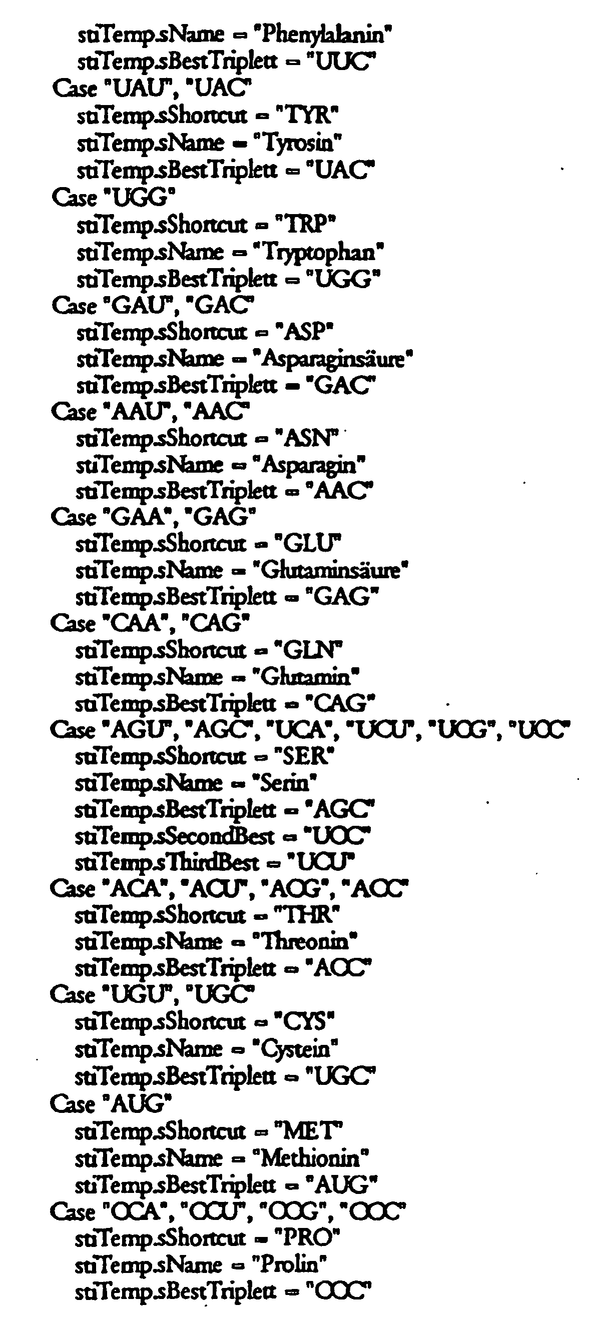

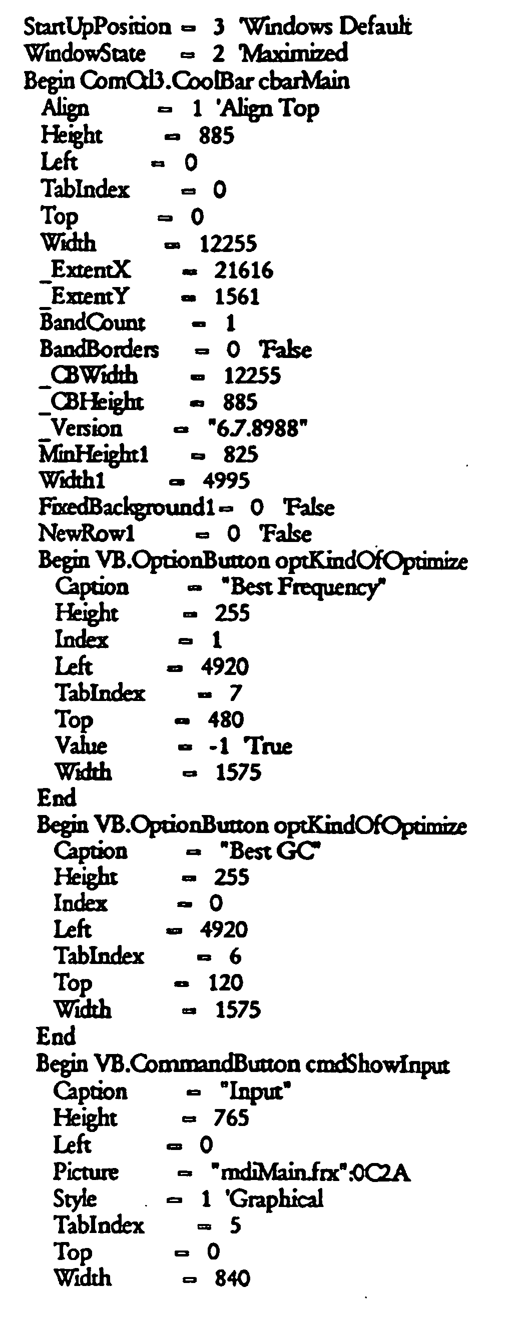

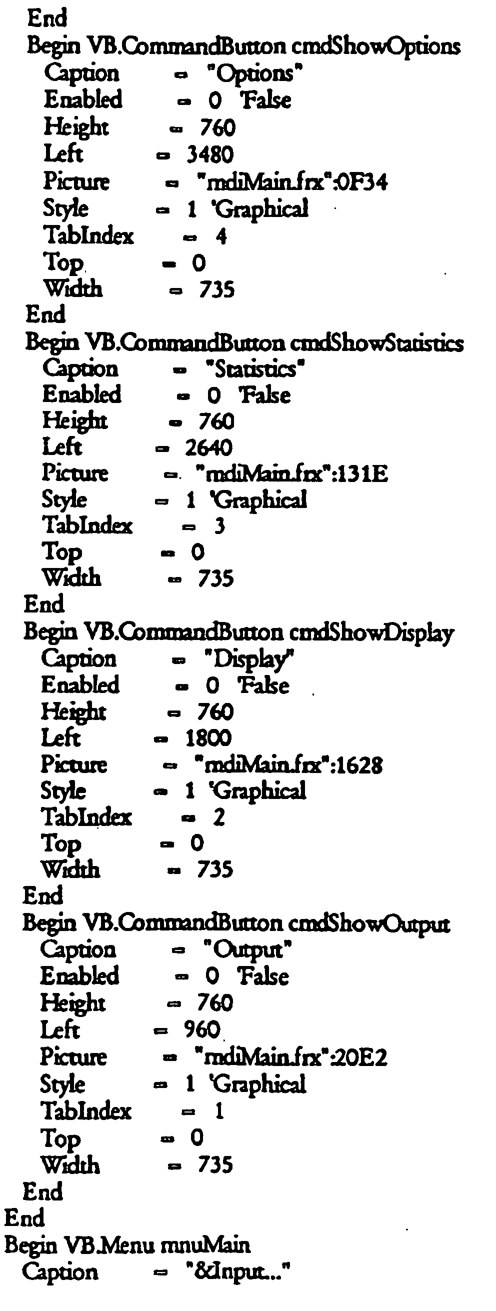

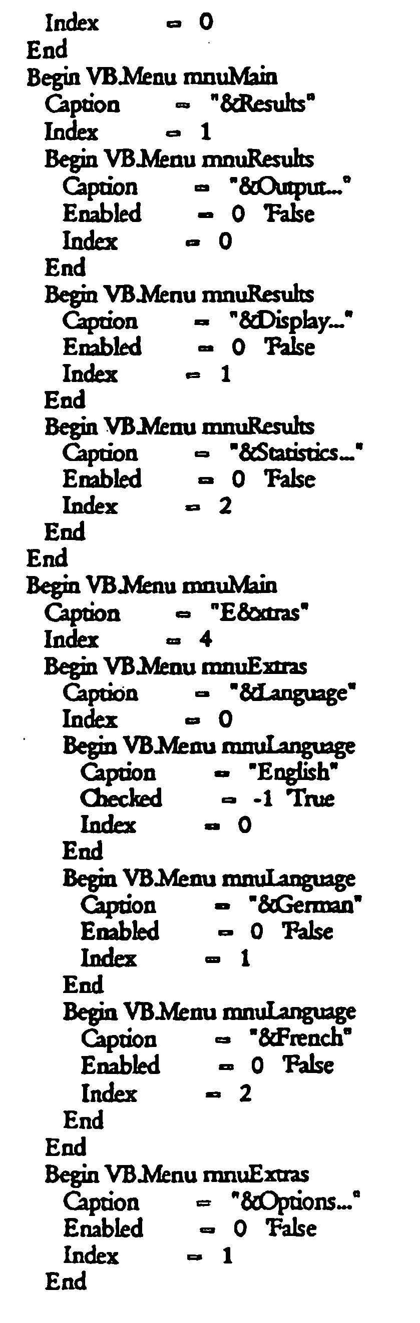

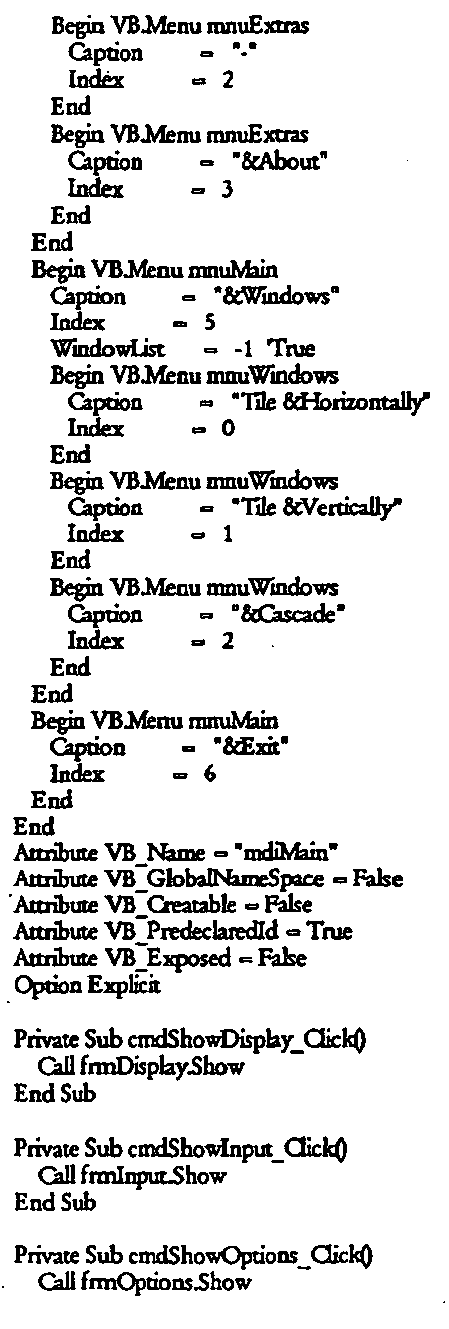

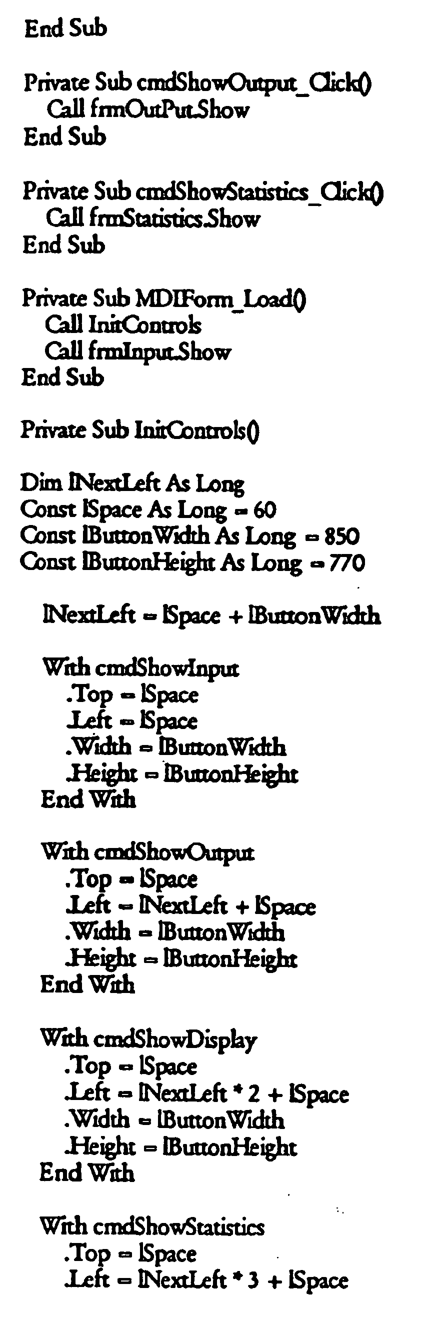

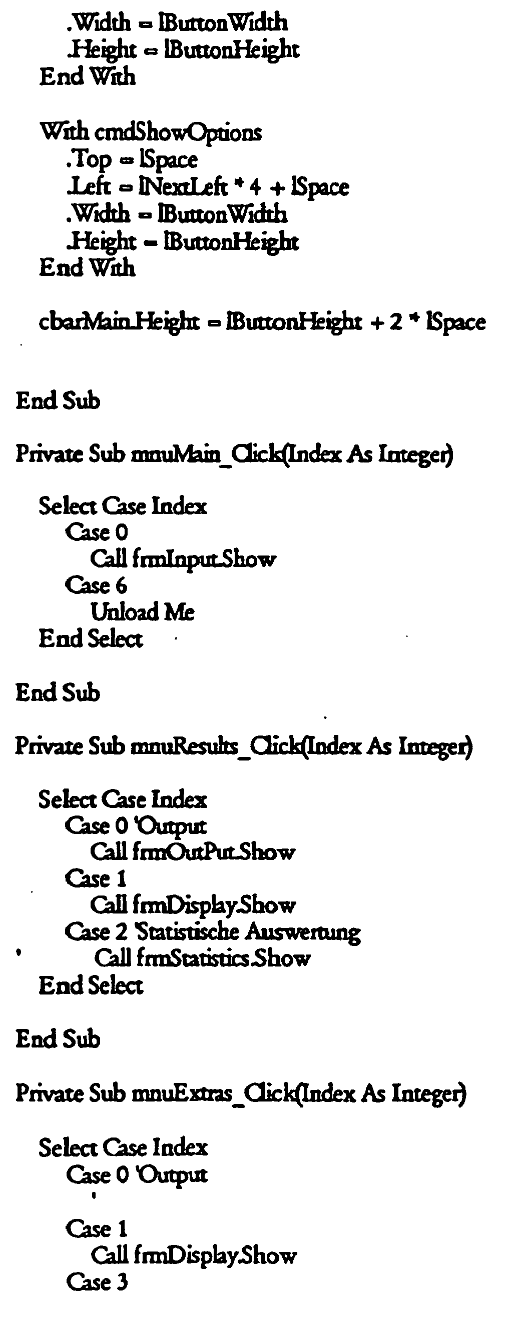

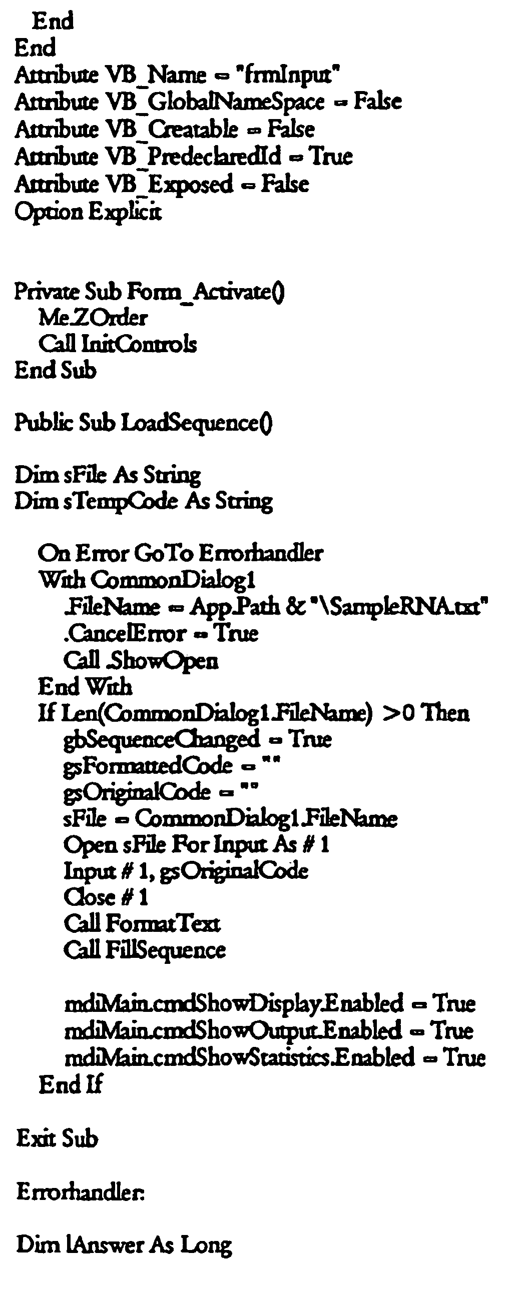

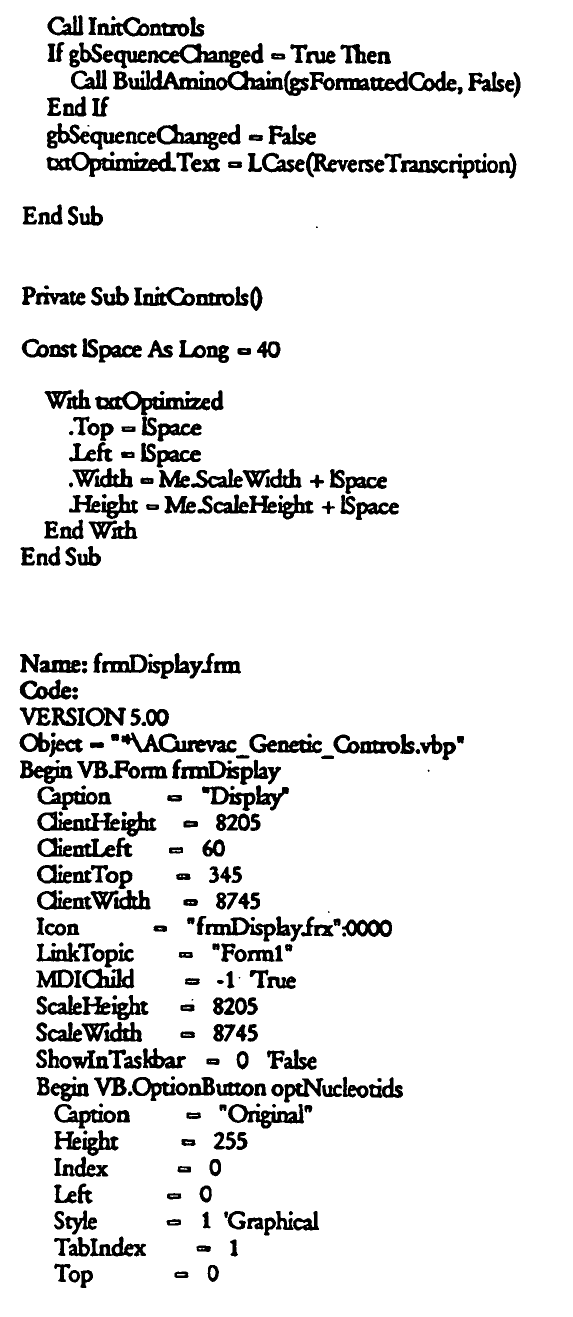

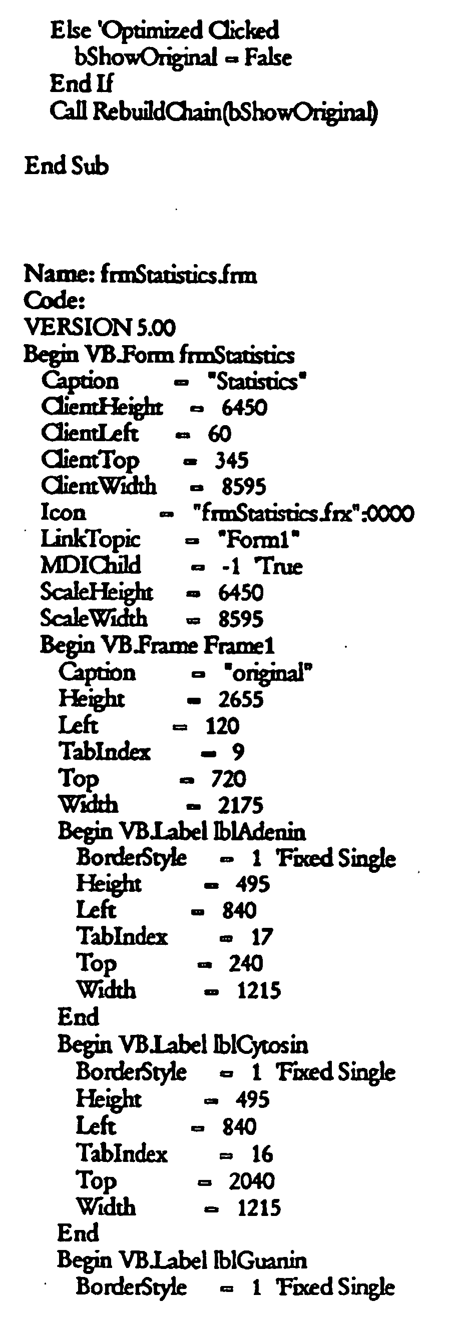

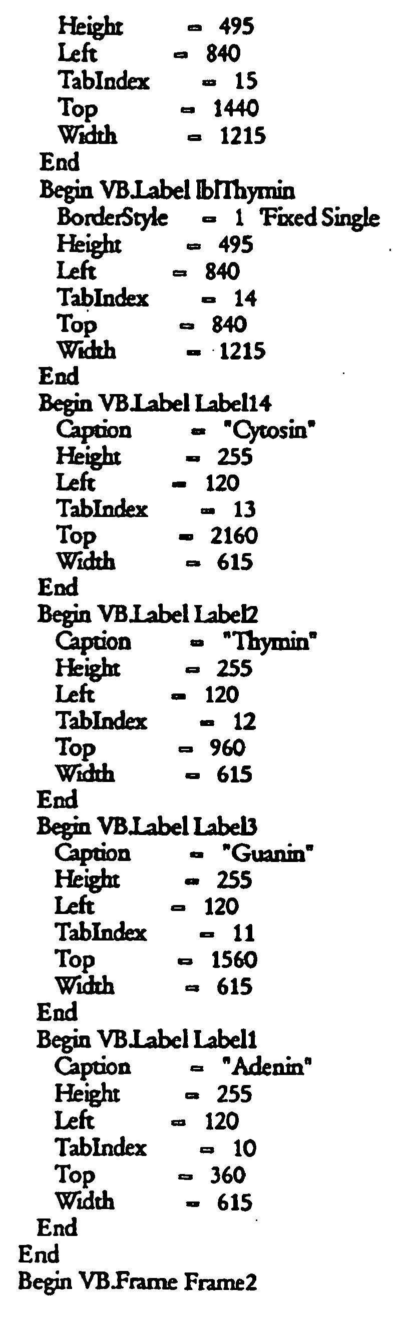

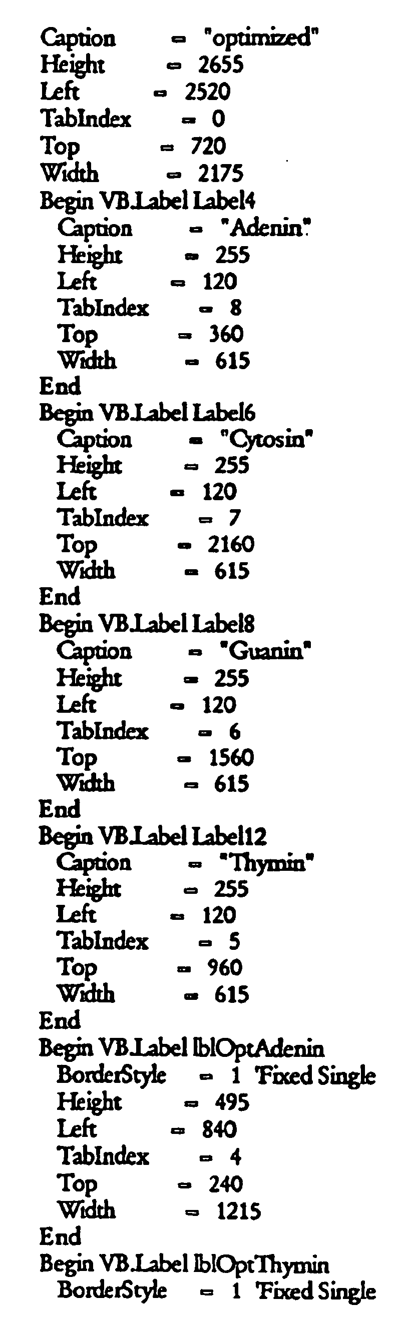

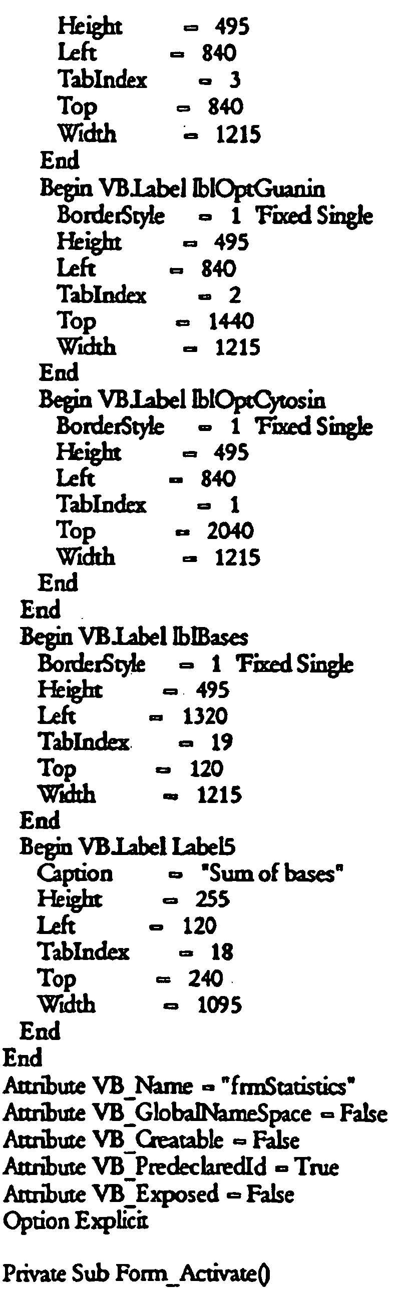

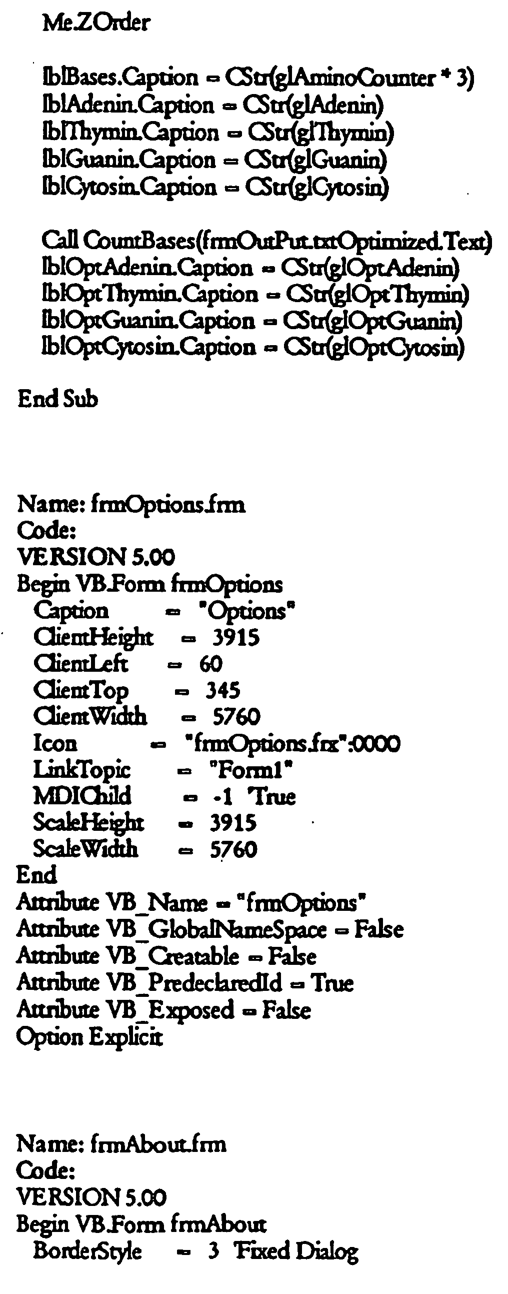

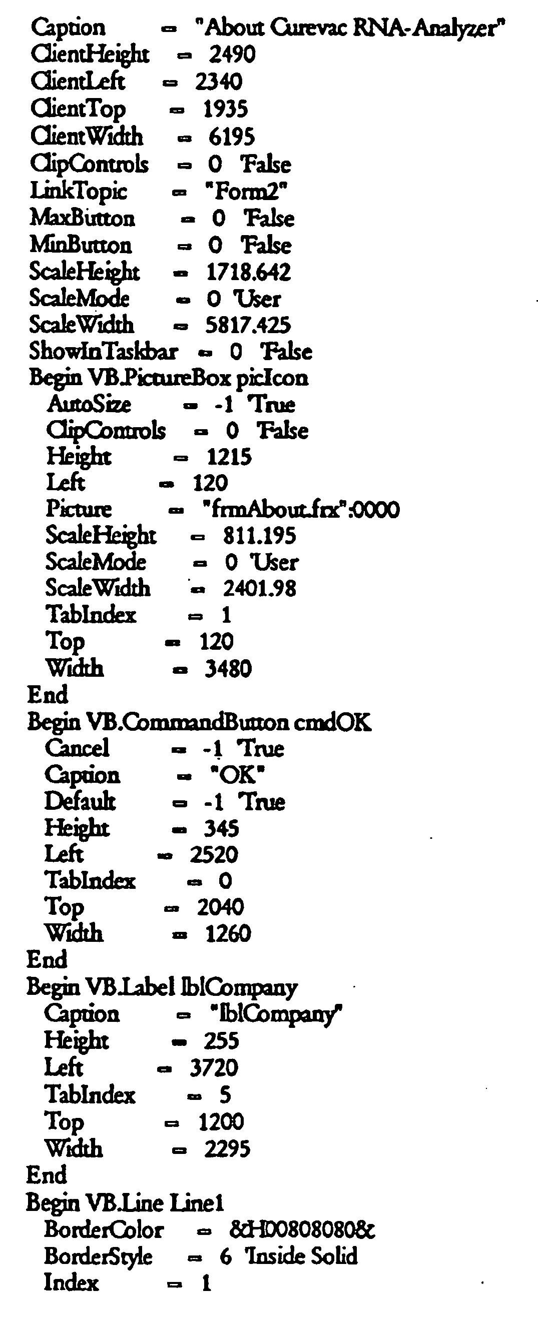

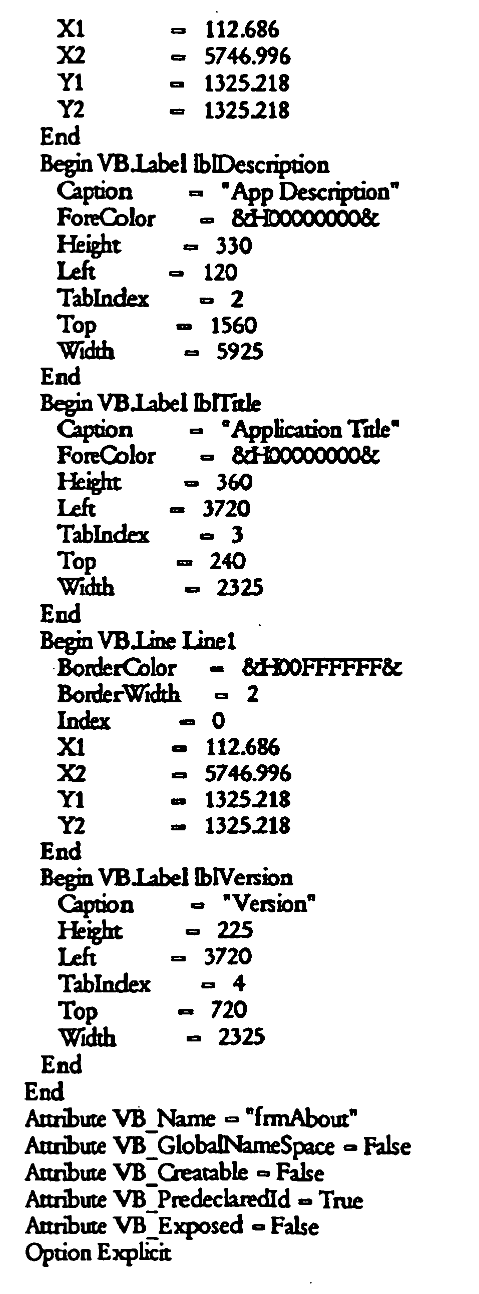

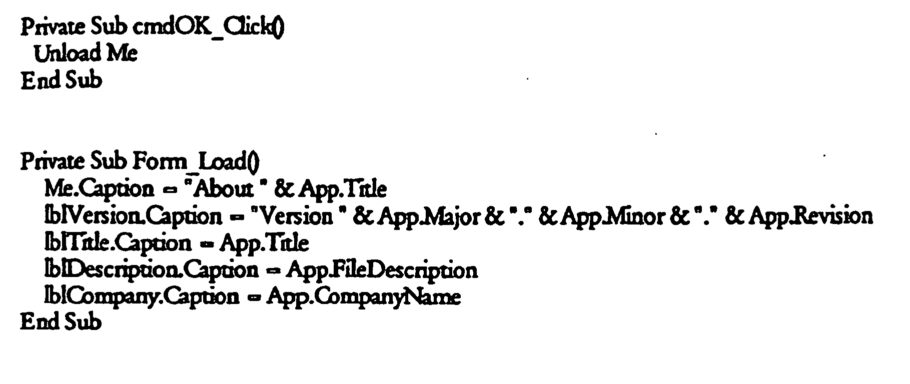

## Patentansprüche

1. Modifizierte mRNA, die für mindestens ein tumorantigenes Peptid oder Polypeptid kodiert, **dadurch gekennzeichnet, dass** der G/C-Gehalt des für das Peptid oder Polypeptid kodierenden Bereichs der modifizierten mRNA größer als der G/C-Gehalt des kodierenden Bereichs der für das Peptid oder Polypeptid kodierenden Wildtyp-mRNA ist und die kodierte Aminosäuresequenz gegenüber dem Wildtyp unverändert ist.

2. Modifizierte mRNA nach Anspruch 1, **dadurch gekennzeichnet, dass** der G/C-Gehalt des für das Peptid oder Polypeptid kodierenden Bereichs der modifizierten mRNA um mindestens 7%-Punkte, bevorzugt mindestens 15%-Punkte, größer als der G/C-Gehalt des kodierenden Bereichs der für das Peptid oder Polypeptid kodierenden Wildtyp-mRNA ist.

3. Modifizierte mRNA nach einem der Ansprüche 1 bis 2, **dadurch gekennzeichnet, dass** die modifizierte mRNA eine 5'-Cap-Struktur und/oder einen polyA-Schwanz von mindestens 70 Nukleotiden und/oder eine IRES und/oder eine 5'-Stabilisierungssequenz und/oder eine 3'-Stabilisierungssequenz aufweist.

4. Modifizierte mRNA nach einem der Ansprüche 1 bis 3, **dadurch gekennzeichnet, dass** die modifizierte mRNA mindestens ein Analoges natürlich vorkommender Nukleotide aufweist.

5. Modifizierte mRNA nach Anspruch 4, **dadurch gekennzeichnet, dass** das Analoge aus der Gruppe, bestehend aus Phosphorthioaten, Phosphoramidaten, Peptidnukleotiden, Methylphosphonaten, 7-Deazaguanosin, 5-Methylcytosin und Inosin, ausgewählt ist.

6. Modifizierte mRNA nach einem der Ansprüche 1 bis 5, **dadurch gekennzeichnet, dass** die mRNA für ein tumorspezifisches Oberflächenantigen kodiert.

7. Modifizierte mRNA nach einem der Ansprüche 1 bis 6, **dadurch gekennzeichnet, dass** die modifizierte mRNA für ein Tumorantigen, ausgewählt aus der Gruppe, bestehend aus 707-AP, AFP, ART-4, BAGE, β-Catenin/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R1701, HPV-E7, HSP70-2M, HAST-2, hTERT (oder hTRT), iCE, KIAA0205, LAGE, LDLR/FUT, MAGE, MART-1/Melan-A, MC1R, Myosin/m, MUC1, MUM-1, -2, -3, NA88-A, NY-ESO-1, p190 minor bcr-abl, Pml/RARα, PRAME, PSA, PSM, RAGE, RU1 oder RU2, SAGE, SART-1 oder SART-3, TEL/AML1, TPI/m, TRP-1, TRP-2, TRP-2/INT2 und WT1, kodiert.

8. Modifizierte mRNA nach einem der Ansprüche 1 bis 7, **dadurch gekennzeichnet, dass** das Polypeptid ein Polyepitop von Tumorantigenen ist.

9. Modifizierte mRNA nach einem der Ansprüche 1 bis 8, **dadurch gekennzeichnet, dass** die mRNA zusätzlich für mindestens ein Cytokin kodiert.

10. Modifizierte mRNA nach einem der Ansprüche 1 bis 9, **dadurch gekennzeichnet, dass** die mRNA mit einem kationischen Peptid oder Protein assoziiert oder daran gebunden ist.

11. Modifizierte mRNA nach Anspruch 10, **dadurch gekennzeichnet, dass** das kationische Peptid oder Protein ausgewählt wird aus der Gruppe bestehend aus Protamin, Poly-L-Lysin und Histonen.

12. Pharmazeutische Zusammensetzung, **dadurch gekennzeichnet, dass** sie modifizierte mRNA nach einem der Ansprüche 1 bis 11 in Verbindung mit einem pharmazeutisch verträglichen Träger und/oder Vehikel enthält.

13. Pharmazeutische Zusammensetzung nach Anspruch 12, **dadurch gekennzeichnet, dass** sie mindestens ein die Immunantwort stimulierendes Adjuvans enthält.

14. Pharmazeutische Zusammensetzung nach einem der Ansprüche 12 oder 13, welche weiter mindestens ein Cytokin enthält.

15. Verwendung einer pharmazeutischen Zusammensetzung nach einem der Ansprüche 12 bis 14 oder einer modifizierten mRNA nach einem der Ansprüche 1 bis 11 zur Herstellung eines Impfstoffs zur Impfung gegen Krebs.

16. Verwendung einer pharmazeutischen Zusammensetzung nach einem der Ansprüche 12 bis 14 zur Herstellung eines Arzneimittels zur Behandlung von Melanom, Kolon-Karzinom, Lymphomen, kleinzelligem Lungenkarzinom Sarkomen und/oder Blastomen.

## Claims

1. Modified mRNA coding for at least one tumour antigenic peptide or polypeptide, **characterised in that** the G/C content of the region of the modified mRNA coding for the peptide or polypeptide is increased compared to the G/C content of the coding region of the wild type mRNA coding for the peptide or polypeptide, and the encoded amino acid sequence is unchanged as compared to the wild type.

2. Modified mRNA according to claim 1, **characterised in that** the G/C content of the region of the modified mRNA coding for the peptide or polypeptide is increased by at least 7 % points, preferably at least 15 % points compared to the G/C content of the coding region of the wild type mRNA coding for the peptide or polypeptide.

3. Modified mRNA according to one of claims 1 to 2, **characterised in that** the modified mRNA comprises a 5' cap structure and/or a poly-A tail of at least 70 nucleotides and/or an IRES and/or a 5' stabilisation sequence and/or a 3' stabilisation sequence.

4. Modified mRNA according to one of claims 1 to 3, **characterised in that** the modified mRNA comprises at least one analogue of naturally occurring nucleotides.

5. Modified mRNA according to claim 4, **characterised in that** the analogue is selected from the group consisting of phosphorus thioates, phosphorus amidates, peptide nucleotides, methylphosphonates, 7-deazaguanosine, 5-methylcytosine, and inosine.

6. Modified mRNA according to one of claims 1 to 5, **characterised in that** the mRNA codes for a tumour specific surface antigen.

7. Modified mRNA according to one of claims 1 to 6, **characterised in that** the modified mRNA codes for a tumour antigen selected from the group consisting of 707-AP, AFP, ART-4, BAGE, ß-catenin/m, Brc-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A*0201-R1701, HPV-E7, HSP70-2M, HAST-2, hTERT (or hTRT), iCE, KIAA0205, LAGE, LDLR/FUT, MAGE, MART-1/melan-A, MC1 R, myosin/m, MUC1, MUM-1, -2, -3, NA88-A, NY-ESO-1, p190 minor bcr-abl, PmI/RARa, PRAME, PSA, PSM, RAGE, RU1 or RU2, SAGE, SART-1 or SART-3, TEL/AML1, TPI/m, TRP-1, TRP-2, TRP-2/INT2 and WT1.

8. Modified mRNA according to one of claims 1 to 7, **characterised in that** the polypeptide is a polyepitope of tumour antigens.

9. Modified mRNA according to one of claims 1 to 8, **characterised in that** the mRNA additionally codes for at least one cytokine.

10. Modified mRNA according to one of claims 1 to 9, **characterised in that** the mRNA is associated with a cationic peptide or protein or is bound thereto.

11. Modified mRNA according to claim 10, **characterised in that** the cationic peptide or protein is selected from the group consisting of protamine, poly-L lysine and histones.

12. Pharmaceutical composition, **characterised in that** the pharmaceutical composition contains modified mRNA according to one of claims 1 to 11 in combination with a pharmaceutically acceptable carrier and/or vehicle.

13. Pharmaceutical composition according to claim 12, **characterised in that** the pharmaceutical composition contains at least one the immune response stimulating adjuvant.

14. Pharmaceutical composition according to one of claims 12 or 13, which in addition contains at least one cytokine.

15. Use of a pharmaceutical composition according to one of claims 12 to 14 or a modified mRNA according to one of claims 1 to 11 for the preparation of a vaccine for inoculation against cancer.

16. Use of a pharmaceutical composition according to one of claims 12 to 14 for the preparation of a medicament for the treatment of melanoma, colon carcinoma, lymphoma, small-cell lung cancer, sarcoma and/or blastoma.

## Revendications

1. ARNm modifié qui code pour au moins un peptide ou polypeptide antigène de tumeurs, **caractérisé en ce que** la teneur en G/C de la région de l'ARNm modifié codant pour le peptide ou polypeptide est augmentée par rapport à la teneur en G/C de la région codante de l'ARNm de type sauvage codant pour le peptide ou polypeptide, et **en ce que** la séquence d'acide aminés codée est inchangée par rapport au type sauvage.

2. ARNm modifié selon la revendication 1, **caractérisé en ce que** la teneur en G/C de la région de l'ARNm modifié codant pour le peptide ou polypeptide est augmentée d'au moins 7 points, préféré d'au moins 15 points, en pourcentage par rapport à la teneur en G/C de la région codante de l'ARNm de type sauvage codant pour le peptide ou polypeptide.

3. ARNm modifié selon l'une des revendications 1 à 2, **caractérisé en ce que** l'ARNm modifié comporte une structure de coiffe 5' et/ou une queue poly-A d'au moins 70 nucleotides et/ou une séquence IRES et/ou une séquence de stabilisation 5' et/ou une séquence de stabilisation 3'.

4. ARNm modifié selon l'une des revendications 1 à 3, **caractérisé en ce que** l'ARNm modifié comporte au moins un analogue de nucléotides se rencontrant dans la nature.

5. ARNm modifié selon la revendication 4, **caractérisé en ce que** l'analogue est choisi dans le groupe consistant en des phosphothioates, des phosphoamidates, des nucléotides peptidiques, des phosphonates de méthyle, la 7-déazaguanosine, la 5-méthylcytosine et l'inosine.

6. ARNm modifié selon l'une des revendications 1 à 5, **caractérisé en ce que** l'ARNm code pour un antigène de surface spécifique à la tumeur.

7. ARNm modifié selon l'une des revendications 1 à 6, **caractérisé en ce que** l'ARNm modifié code pour un antigène de tumeurs choisi dans le groupe consistant en 707-AP, AFP, ART-4, BAGE, β-caténine/m, Bcr-abl, CAMEL, CAP-1, CASP-8, CDC27/m, CDK4/m, CEA, CT, Cyp-B, DAM, ELF2M, ETV6-AMI1, G250, GAGE, GnT-V, Gp100, HAGE, HER-2/neu, HLA-A*0201-R1701, HPV-E7, HSP70-2M, HAST-2, hTERT (ou hTRT), iCE, KIAA0205, LAGE, LDLR/FUT, MAGE, MART-1/mélan-A, MC1 R, myosine/m, MUC1, MUM-1, -2, -3, NA88-A, NY-ESO-1, p190 minor bcr-abl, Pml/RARa, PRAME, PSA, PSM, RAGE, RU1 ou RU2, SAGE, SART-1 ou SART-3, TEL/AML1, TPI/m, TRP-1, TRP-2, TRP-2/INT2 et WT1.

8. ARNm modifié selon l'une des revendications 1 à 7, **caractérisé en ce que** le polypeptide est un polyépitope d'antigènes de tumeurs.

9. ARNm modifié selon l'une des revendications 1 à 8, **caractérisé en ce que** l'ARNm code en outre pour au moins une cytokine.

10. ARNm modifié selon l'une des revendications 1 à 9, **caractérisé en ce que** l'ARNm est associée à un peptide ou une protéine cationique, ou est lieé à ces derniers.

11. ARNm modifié selon la revendication 10, **caractérisé en ce que** le peptide ou protéine cationique et choisi dans le groupe consistant en la protamine, poly-L-lysine, ou les histones.

12. Composition pharmaceutique, **caractérisée en ce qu'**elle contient de l'ARNm modifié selon une des revendications 1 à 11 combiné à un support et/ou à un véhicule pharmacologiquement compatible.

13. Composition pharmaceutique selon la revendication 12, **caractérisée en ce qu'**elle contient au moins un adjuvant stimulant la réponse immunitaire.

14. Composition pharmaceutique selon l'une des revendications 12 ou 13, qui contient en outre au moins une cytokine.

15. Utilisation d'une composition pharmaceutique selon l'une des revendications 12 à 14 ou d'un ARNm modifié selon l'une des revendications 1 à 11 pour la préparation d'un vaccin destiné à la vaccination contre le cancer.

16. Utilisation d'une composition pharmaceutique selon l'une des revendications 12 à 14 pour la préparation d'un médicament destiné au traitement du mélanome, du carcinome du côlon, des lymphomes, du carcinome pulmonaire à petites cellules, des sarcomes et/ou des blastomes.
